(19) Europäisches Patentamt / European Patent Office / Office européen des brevets

(11) **EP 4 505 992 A1**

(12) **EUROPEAN PATENT APPLICATION**
published in accordance with Art. 153(4) EPC

(43) Date of publication:
**12.02.2025 Bulletin 2025/07**

(21) Application number: **23781120.3**

(22) Date of filing: **01.04.2023**

(51) International Patent Classification (IPC):
*A61K 6/884* (2020.01)    *A61K 6/15* (2020.01)
*A61K 6/16* (2020.01)    *A61K 6/17* (2020.01)
*A61K 6/831* (2020.01)    *A61K 6/84* (2020.01)

(52) Cooperative Patent Classification (CPC):
**A61K 6/15; A61K 6/16; A61K 6/17; A61K 6/831;
A61K 6/84; A61K 6/884**

(86) International application number:
**PCT/JP2023/013748**

(87) International publication number:
**WO 2023/191112 (05.10.2023 Gazette 2023/40)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **01.04.2022 JP 2022062165**

(71) Applicant: **Kuraray Noritake Dental Inc.
Kurashiki-shi, Okayama 710-0801 (JP)**

(72) Inventors:
• **KAJIKAWA, Tatsuya
Tainai-shi, Niigata 959-2653 (JP)**
• **HORIGUCHI, Hirotaka
Tainai-shi, Niigata 959-2653 (JP)**

(74) Representative: **D Young & Co LLP
3 Noble Street
London EC2V 7BQ (GB)**

(54) **DENTAL CURABLE COMPOSITION HAVING FAVORABLE COLOR COMPATIBILITY**

(57) The present invention provides a dental curable composition that demonstrates favorable color compatibility with a wide range of natural tooth shades using a single formulation, even for Class II cavities about 4 mm deep, including those in molars with proximal surfaces. The present invention relates to a dental curable composition comprising a polymerizable monomer (A), a polymerization initiator (B), and a filler (C), wherein the dental curable composition shows a color difference ΔE* of 6.0 or less as calculated by the formula [1] below in both a central portion and a proximal portion of artificial molars of A1 and A4 shades when filled in bulk and cured in Class II cavities of 4.0 mm depth formed in the artificial molars.

$$\Delta E^* = ((L^*_1 - L^*_0)^2 + (a^*_1 - a^*_0)^2 + (b^*_1 - b^*_0)^2)^{1/2} \qquad [1]$$

(Description of the symbols in the formula is omitted.)

EP 4 505 992 A1

FIG.1

**Description**

TECHNICAL FIELD

The present invention relates to dental materials that can replace a part or all of natural teeth in the field of dentistry, particularly a dental curable composition that can be suitably used in applications such as dental composite resins.

BACKGROUND ART

Before the advent of dental curable compositions, particularly dental composite resins, the filling treatment of dental caries primarily involved the use of amalgam restorations, and inlay restorations with materials such as gold alloys.
In comparison, dental composite resins have quickly become popular due to their affordability and the ability to relatively easily achieve shades close to natural teeth. Light-curing dental composite resins, in particular, have become the mainstream for filling restorations because of their easy procedures.
Recently, with advancements in the mechanical strength of dental composite resins and the enhanced adhesive strength of dental bonding agents, light-curing dental composite resins are now being used not only for front teeth but also for areas that experience high occlusal pressure such as molars.
Moreover, in view of minimal intervention (MI), composite resin restorations are advised for Class I and Class II cavity repairs in molars (Non-Patent Document 1).
In the past, when using light-curing dental composite resin for dental cavities, the thickness of the filled layer per exposure to light was generally limited to about 2 mm due to considerations such as the curing depth of dental composite resin related to light exposure. This has limited the use of light-curing dental composite resin to cavities with depths of 2 mm or less.
On the other hand, for deep dental cavities exceeding 2 mm, it has been common practice to repeat the filling and photoirradiation of dental composite resin multiple times. A new category of dental composite resin that has gained interest in recent years from the perspective of reducing treatment time in restorative treatment is "bulk-fill" composite resin, which enables curing of filled layers of about 4 mm thickness by single exposure to light.
The shade of natural teeth differs from person to person, and even the teeth of the same individual have different shades in different parts of teeth. When using dental composite resin to achieve a restoration with high aesthetic quality, it has accordingly been necessary to prepare multiple dental composite resins in different shades, and choose one that best matches the shade of actual teeth. Choosing the best shade is a highly skillful technique, and it is not easy to match shades. From a dentists' perspective, there is a need to keep stock of dental composite resins of different shades, and this is a cost disadvantage.
Recently, there has been research into dental composite resins that can match a broad spectrum of natural tooth shades using just one shade. For instance, Patent Document 1 proposes a dental curing composition that produces interference light (structural coloration) by using inorganic spherical fillers with a narrow particle size distribution and polymerizable monomers that show a lower refractive index upon polymerization compared to these fillers.
Patent Document 1 explains that when the cured product of this dental curable composition is filled into a tooth, the intensity of the interference light produced depends on the chromaticity of the underlying tooth, allowing the composition to match a wide range of tooth colors with a single paste. A commercial dental composite resin believed to utilize this technology is Omnichroma (manufactured by Tokuyama Dental Corporation).

CITATION LIST

Non Patent Literature

Non Patent Literature 1: Guidelines for Dental Caries Treatment, 2nd Edition (Edited by The Japanese Society of Conservative Dentistry, Specified Non-Profit Organization) pp.139-152

Patent Literature

Patent Literature 1: WO2018/194032

## SUMMARY OF INVENTION

Technical Problem

However, based on the research conducted by the present inventors, it was found that the color compatibility of the aforementioned commercial dental composite resin has areas for further improvement when filling certain types of cavities.
Specifically, while it performs well in color compatibility for cavities about 2 mm deep and deep Class I cavities about 4 mm deep, its color compatibility diminishes in deep Class II cavities with a depth of about 4 mm.

This is believed to be due to the smaller area of the underlying tooth in Class II cavities. Specifically, while Class I cavities are surrounded by cavity walls on four sides, Class II cavities are surrounded by cavity walls on three sides. In Class II cavities, this smaller area of the underlying tooth appears to weaken the effect of underlying chromaticity on the product. Additionally, as the cavity depth reaches about 4 mm, there is a decreased likelihood of external light reflecting off the underlying surface. This, along with the reduced underlying area, potentially inhibits the development of color compatibility due to various factors interacting together.

As discussed above, in deep Class II cavities about 4 mm deep, specific challenges arise from factors such as cavity structure and area, making it difficult to anticipate reflection and achieve good color compatibility.

The present invention was made to overcome the issues of related art, and an object of the invention is to provide a dental curable composition that demonstrates favorable color compatibility with a wide range of natural tooth shades using a single formulation, even for Class II cavities about 4 mm deep, including those in molars with proximal surfaces.

Solution to Problem

The present inventors conducted intensive studies to find a solution to the foregoing issues, and found a dental curable composition that demonstrates favorable color compatibility with a wide range of natural tooth shades using just one formulation, even for deep Class II cavities about 4 mm deep. This led to the completion of the present invention after further examinations.

The present invention includes the following.

[1] A dental curable composition comprising a polymerizable monomer (A), a polymerization initiator (B), and a filler (C),
wherein the dental curable composition shows a color difference ΔE* of 6.0 or less as calculated by the formula [1] below in both a central portion and a proximal portion of artificial molars of A1 and A4 shades when filled in bulk and cured in Class II cavities of 4.0 mm depth formed in the artificial molars,

$$\Delta E^* = ((L^*_1 - L^*_0)^2 + (a^*_1 - a^*_0)^2 + (b^*_1 - b^*_0)^2)^{1/2} \qquad [1]$$

where $L^*_1$, $a^*_1$, and $b^*_1$ represent the lightness (L value) and chromaticity (a value and b value) in the central or proximal portion after the dental curable composition is filled in bulk and cured in the Class II cavities, and $L^*_0$, $a^*_0$, and $b^*_0$ represent the lightness (L value) and chromaticity (a value and b value) in the central or proximal portion of untreated artificial molars before Class II cavity formation as measured at the same locations as for $L^*_1$, $a^*_1$, and $b^*_1$.
[2] The dental curable composition according to [1], which has a form retention of 90% or more at 37°C in paste form as calculated by the formula below when formed into a cylindrical molded body with a diameter of 8 mm and a height of 10 mm on a glass plate, and the height h [mm] of the cylindrical molded body is measured after being left to stand at 37°C for 1 minute,

$$\text{form retention (\%)} = (h/10) \times 100$$

[3] The dental curable composition according to [1] or [2], wherein a 0.25 mm thick sample plate made of a cured product of the dental curable composition satisfies a light diffusivity LD of 0.0001 to 0.75 as defined by the following formula [2],

$$LD = (I_5/\cos 5°)/I_0 \qquad [2]$$

where I represents the luminous intensity of light transmitted through the sample plate, and $I_0$ and $I_5$ represent the luminous intensities of transmitted light at 0- and 5-degree angles, respectively, with respect to a direction perpendicular to the sample plate (the direction of light incidence).

[4] The dental curable composition according to any one of [1] to [3], which further comprises a colorant (D) (the colorant (D) having a refractive index higher than 2.00 at 25°C when the colorant (D) is an inorganic pigment).

[5] The dental curable composition according to any one of [1] to [4], wherein the filler (C) comprises at least one filler selected from the group consisting of the following fillers (C1), (C2), (C3), and (C4),

filler (C1): a filler having an average particle diameter of 0.001 $\mu$m or more and less than 0.1 $\mu$m, and containing at least one metal (M) selected from the group consisting of aluminum, titanium, strontium, zirconium, barium, lanthanum, and ytterbium;

filler (C2): a filler having an average particle diameter of 1 $\mu$m or more and 50 $\mu$m or less, and containing 5 mass% or more of the metal (M);

filler (C3): a light-diffusing filler having an average particle diameter of 1 $\mu$m or more and 50 $\mu$m or less, and containing 0 mass% or more and less than 5 mass% of the metal (M);

filler (C4): a filler having an average particle diameter of 0.1 $\mu$m or more and less than 1 $\mu$m.

[6] The dental curable composition according to [5], wherein the filler (C) comprises at least two fillers selected from the group consisting of the fillers (C1), (C2), (C3), and (C4).

[7] The dental curable composition according to [5] or [6], wherein the filler (C) comprises the filler (C2) and/or the filler (C3).

[8] The dental curable composition according to any one of [5] to [7], wherein the filler (C) comprises the filler (C1).

[9] The dental curable composition according to any one of [5] to [7], wherein the filler (C2) is an inorganic agglomerated particle (C2-1) formed by agglomeration of inorganic primary particles (x), or an organic-inorganic composite filler (C2-2) containing inorganic primary particles (x), and the inorganic primary particles (x) have an average particle diameter of 0.001 $\mu$m or more and less than 1 $\mu$m.

[10] The dental curable composition according to any one of [5] to [7], wherein the filler (C3) is an inorganic agglomerated particle (C3-1) formed by agglomeration of inorganic primary particles (x), or an organic-inorganic composite filler (C3-2) containing inorganic primary particles (x), and the inorganic primary particles (x) have an average particle diameter of 0.001 $\mu$m or more and less than 1 $\mu$m.

[11] The dental curable composition according to any one of [5] to [10], wherein the filler (C) comprises all of the fillers (C1), (C2), (C3), and (C4).

[12] The dental curable composition according to any one of [5] to [11], wherein the total content of the fillers (C1), (C2), (C3), and (C4) is 10 to 97 mass%.

[13] The dental curable composition according to any one of [5] to [12], wherein the fillers (C1), (C2), (C3), and (C4) satisfy the following formulae [I-1], [I-2], [I-3], and [I-4], respectively,

$$0 \leq nC1\text{-}nA \leq 0.2 \qquad [\text{I-1}]$$

$$-0.03 \leq nC2\text{-}nA \leq 0.03 \qquad [\text{I-2}]$$

$$-0.2 \leq nC3\text{-}nA \leq 0 \qquad [\text{I-3}]$$

$$-0.035 \leq nC4\text{-}nA \leq 0.035 \qquad [\text{I-4}]$$

where nA represents the refractive index of the polymerizable monomer (A) at 25°C, ranging from 1.35 to 1.70, and nC1, nC2, nC3, and nC4 represent the refractive indices of the fillers (C1), (C2), (C3), and (C4), respectively, at 25°C, ranging from 1.30 to 2.00.

Advantageous Effects of Invention

According to the present invention, a dental curable composition can be provided that demonstrates favorable color compatibility with a wide range of natural tooth shades using a single formulation, even for Class II cavities about 4 mm deep, including those in molars with proximal surfaces.

According to the present invention, a dental curable composition can be provided that demonstrates favorable color compatibility with a wide range of natural tooth shades, even for Class I cavities about 4 mm deep.

Furthermore, by employing embodiments where the dental curable composition exhibits light-diffusing properties, the color tone of the dental curable composition in the filled portion can blend naturally with the surroundings, making the filled portion less noticeable, providing a dental curable composition having excellent aesthetic qualities, even in other classes of cavities, including Class III and IV.

A dental curable composition of the present invention can be suitably used for dental composite resins. With the present invention, a dental curable composition can be provided that has a deep depth of cure of 4 mm or more with excellent handling properties.

The present invention can also provide a dental curable composition that exhibits excellent form retention.

BRIEF DESCRIPTION OF DRAWINGS

[FIG. 1] A photograph depicting the formation site of a Class II cavity, along with the measurement locations for lightness and chromaticity, as seen from the upper bottom side of an artificial molar according to the method of measurement of $\Delta E^*$ in the present invention.

[FIG. 2] A photograph depicting the formation site of a Class II cavity as seen from the proximal surface side of an artificial molar according to the method of measurement of $\Delta E^*$ in the present invention.

DESCRIPTION OF EMBODIMENTS

The following describes the present invention in detail.

A dental curable composition of the present invention is a dental curable composition comprising a polymerizable monomer (A), a polymerization initiator (B), and a filler (C),

wherein the dental curable composition shows a color difference $\Delta E^*$ of 6.0 or less as calculated by the formula (1) below in both a central portion and a proximal portion of artificial molars of A1 and A4 shades when filled in bulk and cured in Class II cavities of 4.0 mm depth formed in the artificial molars,

$$\Delta E^* = ((L^*_1 - L^*_0)^2 + (a^*_1 - a^*_0)^2 + (b^*_1 - b^*_0)^2)^{1/2} \qquad (1)$$

where $L^*_1$, $a^*_1$, and $b^*_1$ represent the lightness (L value) and chromaticity (a value and b value) in the central or proximal portion after the dental curable composition is filled in bulk and cured in the Class II cavities, and $L^*_0$, $a^*_0$, and $b^*_0$ represent the lightness (L value) and chromaticity (a value and b value) in the central or proximal portion of untreated artificial molars before Class II cavity formation as measured at the same locations as for $L^*_1$, $a^*_1$, and $b^*_1$.

In a dental curable composition of the present invention, a single formulation provides a color difference $\Delta E^*$ of 6.0 or less at two locations (central and proximal portions) in each of artificial molars of two different shades (tones) A1 and A4. That is, the color difference $\Delta E^*$ is 6.0 or less in all four locations.

With such a configuration, a dental curable composition can be provided that demonstrates favorable color compatibility with a wide range of natural tooth shades using a single formulation, even for Class II cavities about 4 mm deep, including those in molars with proximal surfaces.

In this specification, "Class I cavity" and "Class II cavity" follow the Black's classification. The Black's classification refers to a system of categorizing cavities, and includes Class I, Class II, Class III, Class IV, and Class V. Class I cavities are those formed in caries originating in places such as pits and fissures of molars. Class II cavities are those originating in proximal surfaces of molars (surfaces facing adjacent teeth). Class III cavities are those originating in proximal surfaces of front teeth and canines, excluding the incisal angles. Class IV cavities are those originating in proximal surfaces of front teeth and canines, including the incisal angles. Class V cavities are those occurring on the gingival one-third of teeth on the labial (cheek) or lingual (palate) side of the crown. Additionally, in this specification, "bulk filling" refers to not performing photoirradiation more than twice. Bulk filling encompasses situations where the filling operation is paused one or more times (for stepwise filling), and it can also involve completing the filling in a single operation, provided that there is no second photoirradiation before the filling is completed in filling the dental curable composition from a filling container.

To measure the color difference $\Delta E^*$, artificial molars made of hard resin, specifically GC Zen Opal (manufactured by GC JAPAN, Form Number (Mandible): PL16, No. 6 molar) in shades A1 and A4, are used. A1 and A4 represent classifications of shade (tone), with A1 being higher in lightness than A4, making it appear whiter than A4 to the naked eye.

In this specification, the central portion of the artificial molar refers to any central area on the upper bottom of the tooth, within 2 mm from the center of the pits and fissures, but excluding the pits and fissures themselves.

In this specification, the proximal portion of the artificial molar refers to any portion that lies within 3 mm from the proximal surface (the surface where a neighboring tooth would be) toward the center on the upper bottom of the artificial molar, and is at least 3 mm away from the central portion, avoiding the pits and fissures.

The following describes a specific method for measuring color difference $\Delta E^*$.

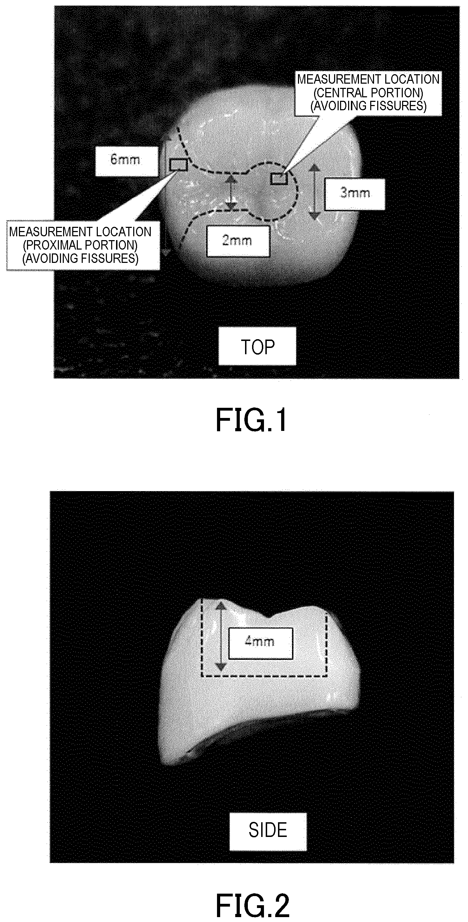

First, a Class II cavity with a depth of 4.0 mm is formed in the artificial molar. The dimensions of the cavity are shown in FIGS. 1 and 2. The cavity can be formed by cutting the artificial tooth with, for example, a micromotor or air turbine equipped with a diamond bur.

Next, an adhesive treatment is applied to the cut surface of the cavity created. Proper adhesive treatment is essential to ensure that the artificial tooth and the dental curable composition bond without separating. If the artificial tooth and the dental curable composition separate, the color of the detached area will change, making it difficult to appropriately evaluate color compatibility. It is preferable to use a dental adhesive to bond the cut surface of the artificial tooth with the dental curable composition. Suitable dental adhesives include, for example, products such as Clearfil® Universal Bond Quick ER (manufactured by Kuraray Noritake Dental Inc.).

In view of providing improved adhesive strength, it is preferable to etch the cut surface of the artificial tooth with a dental etchant before adhesive treatment with a dental adhesive. Suitable dental etchants include, for example, K-Etchant GEL (manufactured by Kuraray Noritake Dental Inc.).

For even higher adhesive strength, it is preferable to use both a dental adhesive and a dental ceramic adhesive for the adhesive treatment. Suitable dental ceramic adhesives include, for example, Clearfil® Porcelain Bond Activator (manufactured by Kuraray Noritake Dental Inc.), and Clearfil® Ceramic Primer Plus (manufactured by Kuraray Noritake Dental Inc.).

Following the adhesive treatment, the dental curable composition is filled into the Class II cavity in bulk, and formed into the occlusal surface shape of the molar. If the dental curable composition is photocurable, this is followed by curing the dental curable composition through exposure to light. The artificial molar, filled with the cured dental curable composition in the Class II cavity measuring 4.0 mm deep, is utilized as a sample for $\Delta E^*$ measurement. The dental curable composition can be filled in bulk using known tools such as spatulas, without any particular limitations.

To shape the occlusal surface of the molar, one method involves, for example, taking an impression of the occlusal surface of the untreated artificial molar (uncut artificial molar) beforehand using a dental clear silicone impression material. This impression can then be pressed onto the filled dental curable composition to give it the shape of the molar occlusal surface. Suitable dental clear silicone impression materials include, for example, Memosil 2 (manufactured by KULZER JAPAN). If the dental curable composition protrudes when the impression is pressed, any excess portion is cleaned off after removing the impression. The impression is placed back in position after removing the excess dental curable composition.

After shaping the molar occlusal surface, a dental visible light irradiator is used to cure the dental curable composition, if it is photocurable. Suitable dental visible light irradiators include, for example, PenCure 2000 (manufactured by J. Morita Corp.). Preferably, light curing is carried out with the impression in position, with light applied over the impression.

If the dental curable composition is a chemically polymerizable curable composition, it must remain with the impression attached until the composition cures completely.

Below is a description of a measurement method for $\Delta E^*$.

First, a dental colorimeter is used to measure the lightness ($L^*_0$) and chromaticity ($a^*_0$, $b^*_0$) of untreated artificial molars before Class II cavity formation.

Suitable dental colorimeters include, for example, the dental colorimeter Crystaleye CE100-DC/JP manufactured by Olympus Corporation under this trade name with a 7-band LED light source and analysis software Crystaleye.

The lightness and chromaticity are measured at the two locations (central portion and proximal portion) shown in FIG. 1.

In this specification, lightness and chromaticity refer to lightness ($L^*$) and chromaticity ($a^*$, $b^*$) according to CIE LAB (JIS Z 8781-4:2013 Color Measurements - Part 4: CIE 1976 L*a*b* color space).

Subsequently, the lightness ($L^*_1$) and chromaticity ($a^*_1$, $b^*_1$) of the filled portion are measured in samples prepared by using the method described above (artificial molars filled with the cured dental curable composition in Class II cavities 4.0 mm deep). The lightness and chromaticity are measured at the two locations (central portion and proximal portion) shown in FIG. 1.

The color difference $\Delta E^*$ is calculated as an indicator of color compatibility, using the following formula (1).

$$\Delta E^* = ((L^*_1 - L^*_0)^2 + (a^*_1 - a^*_0)^2 + (b^*_1 - b^*_0)^2)^{1/2} \qquad (1)$$

where $L^*_1$, $a^*_1$, and $b^*_1$ represent the lightness (L value) and chromaticity (a value and b value) in the central or proximal portion after the dental curable composition is filled in bulk and cured in the Class II cavities, and $L^*_0$, $a^*_0$, and $b^*_0$ represent the lightness (L value) and chromaticity (a value and b value) in the central or proximal portion of untreated artificial molars before Class II cavity formation as measured at the same locations as for $L^*_1$, $a^*_1$, and $b^*_1$.

$\Delta E^*$ is measured at two locations, central and proximal, for samples of each dental curable composition. The same measurement is conducted for artificial molars in shades A1 and A4.

Lower $\Delta E^*$ values are preferred across all four measurements (A1/central, A1/proximal, A4/central, A4/proximal) because it indicates superior color compatibility with a wide range of natural tooth shades when filling in deep cavities formed in natural teeth. The four values of $\Delta E^*$ are 6.0 or less, preferably 5.5 or less, more preferably 5.0 or less, even more preferably 4.8 or less.

Color compatibility for Class II cavities of 4.0 mm depth in artificial molars of shades A1 and A4 can be adjusted by modifying the types and proportions of the polymerizable monomer (A), filler (C), and, optionally, colorant (D) described later. Specifically, for example, when there is a large lightness difference ($|L^*_1-L^*_0|$) in the foregoing measurements, it can be reduced by changing the type of polymerizable monomer (A) or filler (C) and adjusting refractive index, or by increasing and decreasing the colorant (D), particularly a white pigment. As another example, when there is a large chromaticity difference ($|a^*_1-a^*_0|$ or $|b^*_1-b^*_0|$), it can be reduced by increasing and decreasing the colorant (D), particularly a red or yellow pigment.

A dental curable composition of the present invention is expected to enable easier adjustment of the four types of $\Delta E^*$ to 6.0 or less by combining various elements. This includes combining various parameters of filler (C), such as type, average particle size, and content, optionally including a pigment (D), and adjusting variables such as the refractive index difference between polymerizable monomer (A) and filler (C), as well as light diffusivity LD.

Preferably, a dental curable composition of the present invention also has light diffusing properties. The light diffusing properties can be measured with a goniometer or goniophotometer. Specifically, the extent of light diffusing properties can be measured by determining the luminous intensity of diffuse transmitted light relative to specularly transmitted light after the light perpendicularly incident on the cured product of the dental curable composition is measured for the luminous intensity of specularly transmitted light and the luminous intensity of diffuse transmitted light other than the specularly transmitted light. By having a certain degree of light diffusing properties, a dental curable composition of the present invention can produce a blurring effect that blurs the boundaries between natural teeth and the filled material when cavities in natural teeth are filled and restored with a dental curable composition of the present invention. With this effect, the shade of natural teeth can blend in with the portion filled with the dental curable composition in the cavities (hereinafter, also referred to simply as "filled portion"), and the filled portion becomes less noticeable, making it possible to provide a remarkably aesthetic restoration method. This is particularly effective in cases involving restoration of cavities extending from labial side to lingual side, such as in Class III or IV of the Black's classification. In such cases, the restored areas normally show an appearance with a dark, subdued color when a low-contrast-ratio (high-transparency) dental curable composition is used for restoration. Aesthetics can improve with the use of a dental curable composition having an increased contrast ratio (low transparency) provided by, for example, increasing the amount of a pigment such as titanium oxide. However, a dental curable composition of the present invention imparted with light diffusing properties can provide a highly aesthetic natural appearance even with a relatively low contrast ratio (high transparency).

A dental curable composition of the present invention has a light diffusivity LD of preferably 0.0001 or more, more preferably 0.001 or more, even more preferably 0.0015 or more as defined by the formula [2] below for a 0.25 mm thick sample plate made of the cured product of the dental curable composition. The light diffusivity LD may be 0.005 or more, or 0.01 or more. The light diffusivity LD is preferably 0.75 or less, more preferably 0.65 or less, even more preferably 0.6 or less, and may be 0.5 or less, 0.4 or less, or 0.3 or less. The light diffusivity LD is defined by the following formula.

$$LD = (I_5/\cos 5°)/I_0 \qquad [2]$$

where I represents the luminous intensity of light transmitted through the sample plate, and $I_0$ and $I_5$ represent the luminous intensities of transmitted light at 0- and 5-degree angles, respectively, with respect to a direction perpendicular to the sample plate (the direction of light incidence).

In formula [2], the value of luminous intensity at 5° is divided by the cosine of this angle to convert it into a form that conforms to a measure perceivable by human eyes. That is, following the definition of illuminance, the luminous intensity can be converted into illuminance by dividing it by the cosine of the measurement angle. Accordingly, the light diffusing properties are stronger as the LD value approaches 1.

When the cured product of the dental curable composition has a light diffusivity LD within the foregoing lower limit ranges, it is possible to effectively reduce a dark dull impression in the filled portion, or blur the boundaries between the filled portion and natural teeth. When the light diffusivity LD is within the foregoing upper limit ranges, the shade of the cavity floor can effectively manifest in the filled portion. This can lead to even superior color compatibility not only in deep Class I cavities of about 4 mm but also in deep Class II cavities of about 4 mm depth.

The light diffusivity LD can be measured using the method described in the EXAMPLES section below.

The light diffusivity LD can be adjusted by incorporating a filler (C3) having light diffusing properties (described later), and by adjusting the content of filler (C3).

A dental curable composition of the present invention has a form retention of preferably 90% or more, more preferably 92% or more, even more preferably 94% or more at 37°C in its paste form. When the form retention is within these lower limit ranges, it help dentists to shape an appropriate molar occlusal surface on the filled dental curable composition in the patient's mouth, improving aesthetics in term of a shape. It also offers the advantage of shortening the time needed for shape modification, such as when cutting and adjusting the occlusal surface with a diamond bur after curing.

In the treatment of Class II cavities in particular, more paste is needed to fill the cavities, and the process takes longer compared to Class I cavities due to the need to create complex shapes including proximal surfaces. The treatment of Class II cavities thus requires a dental curable composition that exhibits superior form retention during the procedure. When the form retention is below the foregoing lower limits, the dental curable composition filled inside the mouth may sag or deform under gravity, making it difficult to shape an appropriate molar occlusal surface. The specific method for measuring form retention is described below.

The measurement of form retention involves initially molding the dental curable composition under evaluation into a cylindrical shape (8 mm in diameter × 10 mm in height) at ordinary temperature. The molding method is not particularly limited. For example, the dental curable composition can be easily molded by extruding it from a cylinder, as described in the EXAMPLES section below.

Following this, the molded dental curable composition is kept undisturbed for 1 minute in a 37°C environment inside a container such as an open chamber or thermostatic chamber. After this time period, the molded body is removed from the container at ordinary temperature. The height (h [mm]) of the cylindrical molded body is then measured, and form retention is determined by dividing the measured height h by 10, and multiplying it by 100 ((h/10) × 100 [%]).

When the dental curable composition is photocurable, the measurement must be conducted under yellow light or in a dark room to avoid light curing. In the case of a two-pack type dental curable composition, the measurement is conducted after mixing the two components according to the method recommended by the manufacturer (as outlined in the package insert).

Form retention can be adjusted by modifying the types and proportions of the polymerizable monomer (A) and filler (C) described later. Specifically, form retention can improve by reducing the content of polymerizable monomer (A) with respect to the dental curable composition. In view of adjusting the form retention within the foregoing lower limit ranges, the polymerizable monomer (A) is preferably 40 mass% or less, more preferably 35 mass% or less, even more preferably 30 mass% or less in 100 mass% of the dental curable composition.

The form retention of the dental curable composition tends to improve when the polymerizable monomer (A) has a lower content of 2,2,4-trimethylhexamethylenebis(2-carbamoyloxyethyl)dimethacrylate (commonly known as UDMA). Although the exact mechanism is not entirely understood, the observed effect is believed to be related to the affinity between UDMA and the surface treatment agent (for example, such as 3-methacryloyloxypropyltrimethoxysilane) used for the filler (C). In view of adjusting the form retention within the foregoing lower limit ranges, UDMA is preferably 80 parts or less by mass, more preferably 70 parts or less by mass, even more preferably 60 parts or less by mass, particularly preferably 50 parts or less by mass in 100 parts by mass of polymerizable monomer (A).

In view of adjusting the form retention of the dental curable composition within the foregoing lower limit ranges, the filler (C) is preferably irregularly shaped (a crushed shape), rather than spherical or near spherical.

The following describes the components of a dental curable composition of the present invention.

In this specification, the upper limits and lower limits of numeric ranges (for example, ranges of contents of components, ranges of values calculated from components, and ranges of physical properties) can be appropriately combined. For example, the lower limit of a more preferred numeric range may be appropriately combined with the upper limit of an even more preferred numeric range, within the ranges specified in this specification.

[Polymerizable Monomer (A)]

A dental curable composition of the present invention comprises a polymerizable monomer (A). Known polymerizable monomers used for dental curable compositions can be used as polymerizable monomer (A). Particularly preferred for use are radical polymerizable monomers. Examples of such radical polymerizable monomers include esters of unsaturated carboxylic acids such as $\alpha$-cyanoacrylic acid, (meth)acrylic acid, $\alpha$-halogenated acrylic acid, crotonic acid, cinnamic acid, sorbic acid, maleic acid, and itaconic acid; (meth)acrylamide; (meth)acrylamide derivatives; vinyl esters; vinyl ethers; mono-N-vinyl derivatives; and styrene derivatives. The polymerizable monomer (A) may be used alone, or two or more thereof may be used in combination. Preferred among these are esters of unsaturated carboxylic acids, and (meth)acrylamide derivatives, more preferably (meth)acrylic acid esters, and (meth)acrylamide derivatives, even more preferably (meth)acrylic acid esters.

The term "(meth)acryl" used in this specification is intended to be inclusive of both methacryl and acryl. As used herein, "(meth)acrylic monomer" is intended to be inclusive of both (meth)acrylic acid esters and (meth)acrylamide derivatives. Examples of (meth)acrylic acid esters and (meth)acrylamide derivatives are as follows.

(i) Monofunctional (Meth)Acrylic Acid Esters and (Meth)Acrylamide Derivatives

Examples include methyl (meth)acrylate, isobutyl (meth)acrylate, benzyl (meth)acrylate, dodecyl (meth)acrylate, 2-(N,N-dimethylamino)ethyl (meth)acrylate, 2,3-dibromopropyl (meth)acrylate, 3-(meth)acryloyloxypropyltrimethoxysilane, 11-(meth)acryloyloxyundecyltrimethoxysilane, 2-hydroxyethyl (meth)acrylate, 3-hydroxypropyl (meth)acrylate, 4-hydroxybutyl (meth)acrylate, 6-hydroxyhexyl (meth)acrylate, 10-hydroxydecyl (meth)acrylate, propylene glycol mono(meth)acrylate, glycerin mono(meth)acrylate, erythritol mono(meth)acrylate, phenoxyethylene glycol (meth)acrylate, isobornyl (meth)acrylate, 3-phenoxybenzyl (meth)acrylate, N-methylol(meth)acrylamide, N-hydroxyethyl(meth)acrylamide, N,N-bis(hydroxyethyl)(meth)acrylamide, N,N-dimethyl(meth)acrylamide, N,N-diethyl(meth)acrylamide, N,N-di-n-propyl(meth)acrylamide, N-ethyl-N-methyl(meth)acrylamide, (meth)acryloylmorpholine, (meth)acryloyloxydodecylpyridinium bromide, (meth)acryloyloxydodecylpyridinium chloride, (meth)acryloyloxyhexadecylpyridinium bromide, (meth)acryloyloxyhexadecylpyridinium chloride, ethoxylated-o-phenylphenol (meth)acrylate, ethoxylated-m-phenylphenol (meth)acrylate, ethoxylated-p-phenylphenol (meth)acrylate, propoxylated-o-phenylphenol (meth)acrylate, propoxylated-m-phenylphenol (meth)acrylate, propoxylated-p-phenylphenol (meth)acrylate, o-phenoxybenzyl (meth)acrylate, m-phenoxybenzyl (meth)acrylate, p-phenoxybenzyl (meth)acrylate, 2-(o-phenoxyphenyl)ethyl (meth)acrylate, 2-(m-phenoxyphenyl)ethyl (meth)acrylate, and 2-(p-phenoxyphenyl)ethyl (meth)acrylate. In view of good ease of handling of the paste of dental curable composition obtained, and excellence of mechanical strength after cure, most preferred are ethoxylated-o-phenylphenol (meth)acrylate, and m-phenoxybenzyl (meth)acrylate.

(ii) Bifunctional (Meth)Acrylic Acid Esters

Examples include aromatic bifunctional (meth)acrylic acid esters, and aliphatic bifunctional (meth)acrylic acid esters.

Examples of the aromatic bifunctional (meth)acrylic acid esters include 2,2-bis((meth)acryloyloxyphenyl)propane, 2,2-bis[4-(3-acryloyloxy-2-hydroxypropoxy)phenyl]propane, 2,2-bis[4-(2-hydroxy-3-methacryloyloxypropoxy)phenyl]propane (commonly known as Bis-GMA), 2,2-bis(4-(meth)acryloyloxypolyethoxyphenyl)propane, 2-(4-(meth)acryloyloxydiethoxyphenyl)-2-(4-(meth)acryloyloxyethoxyphenyl)propane, 2-(4-(meth)acryloyloxydiethoxyphenyl)-2-(4-(meth)acryloyloxytriethoxyphenyl)propane, 2,2-bis(4-(meth)acryloyloxypropoxyphenyl)propane, 2,2-bis(4-(meth)acryloyloxyisopropoxyphenyl) propane, 2,2-bis(4-(meth)acryloyloxydipropoxyphenyl)propane, 2-(4-(meth)acryloyloxydipropoxyphenyl)-2-(4-(meth)acryloyloxytriethoxyphenyl)propane, 9,9-bis[4-(2-(meth)acryloyloxyethoxy)phenyl] fluorene, 9,9-bis[4-(2-(meth)acryloyloxypolyethoxy)phenyl]fluorene, diphenyl bis[3-(meth)acryloyloxypropyl]silane, and methylphenyl bis[3-(meth)acryloyloxypropyl]silane.

Examples of the aliphatic bifunctional (meth)acrylic acid esters include glycerol di(meth)acrylate, ethylene glycol di(meth)acrylate, diethylene glycol di(meth)acrylate, triethylene glycol diacrylate, triethylene glycol dimethacrylate (commonly known as 3G), propylene glycol di(meth)acrylate, butylene glycol di(meth)acrylate, neopentyl glycol di(meth)acrylate, 1,3-butanediol di(meth)acrylate, 1,5-pentanediol di(meth)acrylate, 1,6-hexanediol di(meth)acrylate, 1,10-decanediol di(meth)acrylate, 1,12-dodecanediol di(meth)acrylate, 1,2-bis(3-(meth)acryloyloxy-2-hydroxypropyloxy)ethane, tricyclodecanedimethanol di(meth)acrylate, 2,2,4-trimethylhexamethylene bis(2-carbamoyloxyethyl)diacrylate, 2,2,4-trimethylhexamethylene bis(2-carbamoyloxyethyl)dimethacrylate (commonly known as UDMA), and dicyclohexyl bis[3-(meth)acryloyloxypropyl]silane.

In view of considerations such as improvement of the ease of handling of the dental curable composition, and improvement of the mechanical strength of the cured product obtained, more preferred as bifunctional (meth)acrylic acid esters are 2,2-bis[4-(3-acryloyloxy-2-hydroxypropoxy)phenyl]propane, 2,2-b is[4-(2-hydroxy-3-m ethacryloyloxypropoxy) phenyl] propane (Bis-GMA), 2,2-bis(4-(meth)acryloyloxypolyethoxyphenyl)propane (average number of moles of ethyleneoxy group added: 1 to 30), triethylene glycol diacrylate, triethylene glycol dimethacrylate (3G), 1,10-decanediol di(meth)acrylate, 1,12-dodecanediol di(meth)acrylate, 1,2-bis(3-(meth)acryloyloxy-2-hydroxypropyloxy)ethane, tricyclodecanedimethanol di(meth)acrylate, 2,2,4-trimethylhexamethylene bis(2-carbamoyloxyethyl)diacrylate, and 2,2,4-trimethylhexamethylene bis(2-carbamoyloxyethyl)dimethacrylate (UDMA), even more preferably 2,2-b is[4-(2-hydroxy-3-m ethacryloyloxypropoxy) phenyl] propane (Bis-GMA), 2,2-bis(4-methacryloyloxypolyethoxyphenyl)propane (average number of moles of ethyleneoxy group added: 1 to 30), triethylene glycol dimethacrylate (3G), 1,10-decanediol dimethacrylate, 1,12-dodecanediol dimethacrylate, 1,2-bis(3-methacryloyloxy-2-hydroxypropyloxy)ethane, tricyclodecanedimethanol dimethacrylate, and 2,2,4-trimethylhexamethylene bis(2-carbamoyloxyethyl)dimethacrylate (UDMA), particularly preferably 2,2-b is[4-(2-hydroxy-3-m ethacryloyloxypropoxy) phenyl] propane (Bis-GMA), 2,2-bis(4-methacryloyloxypolyethoxyphenyl)propane (average number of moles of ethyleneoxy group added: about 2.6), and triethylene glycol dimethacrylate (3G).

(iii) Tri- and Higher-Functional (Meth)Acrylic Acid Esters

Examples include trimethylolpropane tri(meth)acrylate, trimethylolethane tri(meth)acrylate, trimethylolmethane tri(meth)acrylate, pentaerythritol tri(meth)acrylate, pentaerythritol tetra(meth)acrylate, dipentaerythritol tri(meth)acrylate, dipentaerythritol tetra(meth)acrylate, dipentaerythritol penta(meth)acrylate, N,N'-(2,2,4-trimethylhexamethylene)bis [2-(aminocarboxy)propane-1,3-diol]tetra(meth)ac rylate, and 1,7-di(meth)acryloyloxy-2,2,6,6-tetra(meth)acryloyloxymethyl-4-oxaheptane.

It may be preferable that the polymerizable monomer (A) contain a functional monomer capable of imparting adhesive properties to adherends such as tooth structure, metals, and ceramics because a dental curable composition produced with such a functional monomer can exhibit excellent adhesive properties to such materials, among other advantages.

In view of providing excellent adhesive properties to tooth structure and base metals, the functional monomer may be, for example, a polymerizable monomer having a phosphoric acid group, such as 2-(meth)acryloyloxyethyl dihydrogen phosphate, 10-(meth)acryloyloxydecyl dihydrogen phosphate, and 2-(meth)acryloyloxyethylphenyl hydrogen phosphate; or a polymerizable monomer having a carboxylic acid group, such as 11-(meth)acryloyloxyundecane-1,1-dicarboxylic acid, and 4-(meth)acryloyloxyethoxycarbonylphthalic acid. In view of providing excellent adhesive properties to noble metals, the functional monomer may be, for example, 10-mercaptodecyl (meth)acrylate, 6-(4-vinylbenzyl-n-propyl)amino-1,3,5-triazine-2,4-dithione, a thiouracil derivative described in JP H10-1473 A, or a compound having a sulfur element described in JP H11-92461 A. In view of effective adhesion to ceramics, porcelain, and other dental curable compositions, the functional monomer may be, for example, a silane coupling agent such as γ-(meth)acryloyloxypropyltrimethoxysilane.

The content of the polymerizable monomer (A) in a dental curable composition of the present invention is not particularly limited. However, in view of properties such as the handling properties the dental curable composition obtained, and the mechanical strength of the cured product, the content of polymerizable monomer (A) is preferably 1 mass% or more, more preferably 2 mass% or more, even more preferably 5 mass% or more, and may be 8 mass% or more, or 15 mass% or more, based on 100 mass% of the dental curable composition. The content of polymerizable monomer (A) is preferably 70 mass% or less, more preferably 50 mass% or less, even more preferably 40 mass% or less, particularly preferably 30 mass% or less.

The polymerizable monomer (A) in the present invention has a refractive index at 25°C of preferably 1.350 to 1.700, more preferably 1.400 to 1.650, even more preferably 1.430 to 1.600. The refractive index can be appropriately selected within these ranges, and may be 1.43 to 1.65, 1.450 or more, or 1.500 or more, and may be 1.580 or less.

When the refractive index of polymerizable monomer (A) falls within these ranges, the transparency of the dental curable composition increases. This can result in a dental curable composition with high aesthetic quality and a high depth of cure. The refractive index of polymerizable monomer (A) can be measured using the method described in [Refractive Index of Polymerizable Monomer (A)] in the EXAMPLES section below.

[Polymerization Initiator (B)]

A dental curable composition of the present invention comprises a polymerization initiator (B). The polymerization initiator (B) can be selected from polymerization initiators used in industry. Preferred for use are polymerization initiators used in dentistry. Particularly preferred are photopolymerization initiators and chemical polymerization initiators. The polymerization initiator (B) may be used alone, or two or more thereof may be used in appropriate combinations.

Examples of the photopolymerization initiators include (bis)acylphosphine oxides, ketals, α-diketones, and coumarins.

Examples of acylphosphine oxides in the (bis)acylphosphine oxides include 2,4,6-trimethylbenzoyldiphenylphosphine oxide, 2,6-dimethoxybenzoyldiphenylphosphine oxide, 2,6-dichlorobenzoyldiphenylphosphine oxide, 2,4,6-trimethylbenzoylmethoxyphenylphosphine oxide, 2,4,6-trimethylbenzoylphenylphosphine oxide, 2,4,6-trimethylbenzoylethoxyphenylphosphine oxide, 2,3,5,6-tetramethylbenzoyldiphenylphosphine oxide, benzoyl di(2,6-dimethylphenyl) phosphonate, and salts of these (such as sodium salts, potassium salts, and ammonium salts).

Examples of bisacylphosphine oxides include bis(2,6-dichlorobenzoyl)phenylphosphine oxide, bis(2,6-dichlorobenzoyl)-2,5-dimethylphenylphosphine oxide, bis(2,6-dichlorobenzoyl)-4-propylphenylphosphine oxide, bis(2,6-dichlorobenzoyl)-1-naphthylphosphine oxide, bis(2,6-dimethoxybenzoyl)phenylphosphine oxide, bis(2,6-dimethoxybenzoyl)-2,4,4-trimethylpentylphosphine oxide, bis(2,6-dimethoxybenzoyl)-2,5-dimethylphenylphosphine oxide, bis(2,4,6-trimethylbenzoyl)phenylphosphine oxide, bis(2,5,6-trimethylbenzoyl)-2,4,4-trimethylpentylphosphine oxide, and salts of these (such as sodium salts, potassium salts, and ammonium salts).

Preferred among these (bis)acylphosphine oxides are 2,4,6-trimethylbenzoyldiphenylphosphine oxide, 2,4,6-trimethylbenzoylmethoxyphenylphosphine oxide, bis(2,4,6-trimethylbenzoyl)phenylphosphine oxide, and sodium salts of 2,4,6-trimethylbenzoylphenylphosphine oxide.

Examples of the ketals include benzyl dimethyl ketal, and benzyl diethyl ketal.

Examples of α-diketones include diacetyl, benzyl, camphorquinone, 2,3-pentadione, 2,3-octadione, 9,10-phenanthrenequinone, and 4,4'-oxybenzyl, and acenaphthenequinone. Preferred is camphorquinone for its maximum absorption wavelength occurring in the visible light region.

Examples of the coumarin compounds include compounds mentioned in JP H09-3109 A and JP H10-245525 A, such as 3,3'-carbonylbis(7-diethylaminocoumarin), 3-(4-methoxybenzoyl)coumarin, 3-thienoylcoumarin, 3-benzoyl-5,7-dimethoxycoumarin, 3-benzoyl-7-methoxycoumarin, 3-benzoyl-6-methoxycoumarin, 3-benzoyl-8-methoxycoumarin, 3-benzoylcoumarin, 7-methoxy-3-(p-nitrobenzoyl)coumarin, 3-(p-nitrobenzoyl)coumarin, 3,5-carbonylbis(7-methoxycoumarin), 3-benzoyl-6-bromocoumarin, 3,3'-carbonylbiscoumarin, 3-benzoyl-7-dimethylaminocoumarin, 3-benzoylbenzo[f]coumarin, 3-carboxycoumarin, 3-carboxy-7-methoxycoumarin, 3-ethoxycarbonyl-6-methoxycoumarin, 3-ethoxycarbonyl-8-methoxycoumarin, 3-acetylbenzo[f]coumarin, 3-benzoyl-6-nitrocoumarin, 3-benzoyl-7-diethylaminocoumarin, 7-dimethylamino-3-(4-methoxybenzoyl)coumarin, 7-diethylamino-3-(4-methoxybenzoyl)coumarin, 7-diethylamino-3-(4-diethylamino)coumarin, 7-methoxy-3-(4-methoxybenzoyl)coumarin, 3-(4-nitrobenzoyl)benzo[f]coumarin, 3-(4-ethoxycinnamoyl)-7-methoxycoumarin, 3-(4-dimethylaminocinnamoyl)coumarin, 3-(4-diphenylaminocinnamoyl)coumarin, 3-[(3-dimethylbenzothiazol-2-ylidene)acetyl]coumarin, 3-[(1-methylnaphtho[1,2-d]thiazol-2-ylidene)acetyl]coumarin, 3,3'-carbonylbis(6-methoxycoumarin), 3,3'-carbonylbis(7-acetoxycoumarin), 3,3'-carbonylbis(7-dimethylaminocoumarin), 3-(2-benzothiazolyl)-7-(diethylamino)coumarin, 3-(2-benzothiazolyl)-7-(dibutylamino)coumarin, 3-(2-benzoimidazolyl)-7-(diethylamino)coumarin, 3-(2-benzothiazolyl)-7-(dioctylamino)coumarin, 3-acetyl-7-(dimethylamino)coumarin, 3,3'-carbonylbis(7-dibutylaminocoumarin), 3,3'-carbonyl-7-diethylaminocoumarin-7'-bis(butoxyethyl)aminocoumarin, 10-[3-[4-(dimethylamino)phenyl]-1-oxo-2-propenyl]-2,3,6,7-tetrahydro-1,1,7,7-tetramethyl-1H,5H,11H-[1]benzopyrrano[6,7,8-ij]quinolizin-11-one, and 10-(2-benzothiazolyl)-2,3,6,7-tetrahydro-1,1,7,7-tetramethyl-1H,5H,11H-[1]benzopyrrano[6,7,8-ij]quinolizin-11-one.

Particularly preferred among these coumarin compounds are 3,3'-carbonylbis(7-diethylaminocoumarin), and 3,3'-carbonylbis(7-dibutylaminocoumarin).

By using at least one selected from the group of photopolymerization initiators consisting of an (bis)acylphosphine oxide, an α-diketone, and a coumarin compound, a dental curable composition can be obtained that has excellent photocurability both in the visible light region and the near ultraviolet region, and that shows sufficient photocurability regardless of whether the light source used is a halogen lamp, a light emitting diode (LED), or a xenon lamp.

Preferred for use as chemical polymerization initiators are organic peroxides. The organic peroxides used as chemical polymerization initiators are not particularly limited, and may be known organic peroxides. Typical examples of organic peroxides include ketone peroxides, hydroperoxides, diacyl peroxides, dialkyl peroxides, peroxyketals, peroxyesters, and peroxydicarbonates.

Examples of the ketone peroxides include methyl ethyl ketone peroxide, methyl isobutyl ketone peroxide, methyl cyclohexanone peroxide, and cyclohexanone peroxide.

Examples of the hydroperoxides include 2,5-dimethylhexane-2,5-dihydroperoxide, diisopropylbenzene hydroperoxide, cumene hydroperoxide, t-butyl hydroperoxide, and 1,1,3,3-tetramethylbutyl hydroperoxide.

Examples of the diacyl peroxides include acetyl peroxide, isobutyryl peroxide, benzoyl peroxide, decanoyl peroxide, 3,5,5-trimethylhexanoyl peroxide, 2,4-dichlorobenzoyl peroxide, and lauroyl peroxide.

Examples of the dialkyl peroxides include di-t-butyl peroxide, dicumyl peroxide, t-butylcumyl peroxide, 2,5-dimethyl-2,5-di(t-butylperoxy)hexane, 1,3-bis(t-butylperoxyisopropyl)benzene, and 2,5-dimethyl-2,5-di(t-butylperoxy)-3-hexyne.

Examples of the peroxyketals include 1,1-bis(t-butylperoxy)-3,3,5-trimethylcyclohexane, 1,1-bis(t-butylperoxy)cyclohexane, 2,2-bis(t-butylperoxy)butane, 2,2-bis(t-butylperoxy)octane, and 4,4-bis(t-butylperoxy)valeric acid-n-butyl ester.

Examples of the peroxyesters include α-cumyl peroxyneodecanoate, t-butyl peroxyneodecanoate, t-butyl peroxypivalate, 2,2,4-trimethylpentyl peroxy-2-ethylhexanoate, t-amyl peroxy-2-ethylhexanoate, t-butyl peroxy-2-ethylhexanoate, di-t-butyl peroxyisophthalate, di-t-butyl peroxyhexahydroterephthalate, t-butyl peroxy-3,3,5-trimethylhexanoate, t-butyl peroxyacetate, t-butyl peroxybenzoate, and t-butyl peroxymaleic acid.

Examples of the peroxydicarbonates include di(3-methoxybutyl)peroxydicarbonate, di(2-ethylhexyl)peroxydicarbonate, bis(4-t-butylcyclohexyl)peroxydicarbonate, diisopropyl peroxydicarbonate, di-n-propyl peroxydicarbonate, di(2-ethoxyethyl)peroxydicarbonate, and diallyl peroxydicarbonate.

From an overall balance of safety, storage stability, and radical generating potential, preferred among these organic peroxides are diacyl peroxides, more preferably benzoyl peroxide.

The content of the polymerization initiator (B) in a dental curable composition of the present invention is not particularly limited. However, in view of considerations such as the curability of the dental curable composition obtained, the content of polymerization initiator (B) is preferably 0.001 parts or more by mass, more preferably 0.01 parts or more by mass, even more preferably 0.02 parts or more by mass, particularly preferably 0.1 parts or more by mass relative to 100 parts by mass of the total mass of polymerizable monomer (A). For considerations such as possible precipitation from the dental curable composition when the content of polymerization initiator (B) is too high, the content of polymerization initiator (B) is preferably 30 parts or less by mass, more preferably 20 parts or less by mass, even more preferably 15 parts or less by mass, particularly preferably 10 parts or less by mass, or may be 5 parts or less by mass, or 2 parts or less by mass, relative to 100 parts by mass of the total mass of polymerizable monomer (A).

[Filler (C)]

A dental curable composition of the present invention comprises a filler (C).

The overall particle shape of filler (C) is not particularly limited, and the filler (C) may be used in the form of an irregularly shaped powder or a spherical powder. With an irregularly shaped filler (C), a dental curable composition can be obtained that excels in form retention and in the mechanical strength and wear resistance of the cured product. With a spherical filler (C), a dental curable composition can be provided that has smooth, easy-to-spread paste properties with excellent handling properties.

The overall shape of filler (C) can be appropriately selected according to the intended use of the dental curable composition. In view of excellence of form retention and the mechanical strength of the cured product, it is preferable to use an irregularly shaped filler (C).

Preferred examples of filler (C) include those containing various types of glass, preferably with silica as a main component, and, optionally, an oxide of heavy metal, boron, aluminum, or the like. Examples of such glass include glass powders of common compositions, such as fused silica, quartz, soda-lime-silica glass, E glass, C glass, and borosilicate glass (PYREX® glass); and dental glass powders, such as barium glass (GM27884, 8235 manufactured by Schott, and E-2000, E-3000 manufactured by ESSTECH), strontium borosilicate glass (E-4000 manufactured by ESSTECH), lanthanum glass-ceramic (GM31684 manufactured by Schott), and fluoroaluminosilicate glass (GM35429, G018-091, G018-117 manufactured by Schott). Other preferred examples of filler (C) include those containing various types of ceramics, alumina, composite oxides (such as silica-titania, silica-zirconia, and silica-ytterbia), diatomaceous earth, kaolin, clay minerals (such as montmorillonite), activated earth, synthetic zeolite, mica, calcium fluoride, ytterbium fluoride, yttrium fluoride, calcium phosphate, barium sulfate, zirconium oxide, titanium oxide, and hydroxyapatite. The filler (C) may be used alone, or two or more thereof may be used in combination.

Preferred are those containing silica (with a silica content of 5 mass% or more, preferably 10 mass% or more).

Among these, it is preferable to comprise an inorganic filler (such as barium glass, alumina, silica-titania, silica-zirconia, and silica-ytterbia) containing metallic elements having high radiopacity (such as barium, zirconium, aluminum, and ytterbium), or ytterbium fluoride.

The filler (C) of the present invention has a refractive index at 25°C of preferably 1.30 to 2.00, more preferably 1.35 to 1.80, even more preferably 1.40 to 1.70.

When the refractive index of filler (C) falls within these ranges, the transparency of the dental curable composition increases. This can result in a dental curable composition with high aesthetic quality and a high depth of cure. The refractive index of filler (C) can be measured using the method described in the EXAMPLES section below.

The filler (C) of the present invention does not require surface treatment. It is, however, preferable that the filler (C) be surface-treated because surface treatment hydrophobizes the surface of filler (C) and improves the affinity to polymerizable monomer (A), and allows the filler (C) to be contained in increased amounts, along with other advantages. A surface treatment agent can be used for surface treatment. The type of surface treatment agent is not particularly limited, and known surface treatment agents can be used, for example, such as silane coupling agents, organotitanium coupling agents, organozirconium coupling agents, organoaluminum coupling agents, phosphoric acid group-containing polymerizable monomers, and carboxylic acid group-containing polymerizable monomers. The surface treatment agent may be used alone, or two or more thereof may be used in combination. Silane coupling agents are preferred in view of considerations such as the affinity of filler (C) to polymerizable monomer (A), and availability.

The silane coupling agent is preferably a compound represented by the following general formula [Z], though the type of silane coupling agent is not particularly limited.

$$H_2C=CR^1-CO-R^2-(CH_2)_q-SiR^3_pR^4_{(3-p)} \qquad [Z]$$

wherein $R^1$ is a hydrogen atom or a methyl group, $R^2$ is an oxygen atom, a sulfur atom, or $-NR^5-$, where $R^5$ is a hydrogen atom or a C1 to C8 aliphatic group (may be linear, branched, or cyclic), $R^3$ is a hydrolyzable group, $R^4$ is a C1 to C6 hydrocarbon group, p is an integer of 1 to 3, q is an integer of 1 to 13, and the multiple $R^3$ and $R^4$ each may be the same or different.

In the general formula [Z], $R^1$ is a hydrogen atom or a methyl group, preferably a methyl group.

$R^2$ is an oxygen atom, a sulfur atom, or $-NR^5-$, preferably an oxygen atom. $R^5$ represents a hydrogen atom, or a C1 to C8 aliphatic group (may be linear, branched, or cyclic), and the C1 to C8 aliphatic group represented by $R^5$ may be a saturated aliphatic group (such as an alkyl group or a cycloalkylene group (e.g., a cyclohexyl group)), or an unsaturated aliphatic group (such as an alkenyl group, or an alkynyl group). In view of considerations such as availability, ease of production, and chemical stability, preferred are saturated aliphatic groups, more preferably alkyl groups.

Examples of the alkyl groups include methyl groups, ethyl groups, n-propyl groups, isopropyl groups, n-butyl groups, isobutyl groups, sec-butyl groups, tert-butyl groups, n-pentyl groups, isopentyl groups, n-hexyl groups, n-heptyl groups, 2-methylhexyl groups, and n-octyl groups.

Preferably, $R^5$ is a hydrogen atom or a C1 to C4 alkyl group, more preferably a hydrogen atom or a C1 to C3 alkyl group, even more preferably a hydrogen atom.

In the general formula [Z], examples of the hydrolyzable group represented by $R^3$ include alkoxy groups such as methoxy groups, ethoxy groups, and butoxy groups; halogen atoms such as a chlorine atom and a bromine atom; and isocyanate groups.

When a plurality of $R^3$ exists, $R^3$ may be the same or different from one another.

Preferably, $R^3$ is an alkoxy group, more preferably a methoxy group or an ethoxy group, even more preferably a methoxy group.

In the general formula [Z], examples of the C1 to C6 hydrocarbon group represented by $R^4$ include C1 to C6 alkyl groups (may be cyclic), C2 to C6 alkenyl groups (may be cyclic), and C2 to C6 alkynyl groups.

When a plurality of $R^4$ exists, $R^4$ may be the same or different from one another.

Examples of the C1 to C6 alkyl groups include methyl groups, ethyl groups, n-propyl groups, isopropyl groups, n-butyl groups, isobutyl groups, sec-butyl groups, tert-butyl groups, n-pentyl groups, isopentyl groups, neopentyl groups, tert-pentyl groups, n-hexyl groups, cyclopropyl groups, cyclobutyl groups, cyclopentyl groups, and cyclohexyl groups.

Examples of the C2 to C6 alkenyl groups include vinyl groups, allyl groups, 1-methylvinyl groups, 1-propenyl groups, butenyl groups, pentenyl groups, hexenyl groups, cyclopropenyl groups, cyclobutenyl groups, cyclopentenyl groups, and cyclohexenyl groups.

Examples of the C2 to C6 alkynyl groups include ethynyl, 1-propynyl, 2-propynyl, 1-butynyl, 1-methyl-2-propynyl, 2-butynyl, 3-butynyl, 1-pentynyl, 1-ethyl-2-propynyl, 2-pentynyl, 3-pentynyl, 1-methyl-2-butynyl, 4-pentynyl, 1-methyl-3-butynyl, 2-methyl-3-butynyl, 1-hexynyl, 2-hexynyl, 1-ethyl-2-butynyl, 3-hexynyl, 1-methyl-2-pentynyl, 1-methyl-3-pentynyl, 4-methyl-1-pentynyl, 3-methyl-1-pentynyl, 5-hexynyl, and 1-ethyl-3-butynyl.

In the general formula [Z], p is an integer of 1 to 3, preferably 2 or 3, more preferably 3. In the general formula [Z], q is an integer of 1 to 13, preferably an integer of 2 to 12, more preferably an integer of 3 to 11.

Specific examples of the silane coupling agent represented by the general formula [Z] include methacryloyloxymethyltrimethoxysilane, 2-methacryloyloxyethyltrimethoxysilane, 3-methacryloyloxypropyltrimethoxysilane, 4-methacryloyloxybutyltrimethoxysilane, 5-methacryloyloxypentyltrimethoxysilane, 6-methacryloyloxyhexyltrimethoxysilane, 7-methacryloyloxyheptyltrimethoxysilane, 8-methacryloyloxyoctyltrimethoxysilane, 9-methacryloyloxynonyltrimethoxysilane, 10-methacryloyloxydecyltrim ethoxysilane, 11-methacryloyloxyundecyltrimethoxysilane, 12-methacryloyloxydodecyltrimethoxysilane, 13-methacryloyloxytridecyltrimethoxysilane, 11-methacryloyloxyundecyldichloromethylsilane, 11-methacryloyloxyundecyltrichlorosilane, and 12-methacryloyloxydodecyldimethoxymethylsilane. The silane coupling agent may be used alone, or two or more thereof may be used in combination.

In view of availability, preferred among these is 3-methacryloyloxypropyltrimethoxysilane. In view of further improvement of the affinity between polymerizable monomer (A) and filler (C), preferred are 8-methacryloyloxyoctyltrimethoxysilane, 9-methacryloyloxynonyltrimethoxysilane, 10-methacryloyloxydecyltrimethoxysilane, and 11-methacryloyloxyundecyltrimethoxysilane.

Examples of the phosphoric acid group-containing polymerizable monomers include monofunctional phosphoric acid group-containing polymerizable monomers such as 2-(meth)acryloyloxyethyl dihydrogen phosphate, 10-(meth)acryloyloxydecyl dihydrogen phosphate, and 2-(meth)acryloyloxyethylphenyl hydrogen phosphate.

Examples of the carboxylic acid group-containing polymerizable monomers include polymerizable monomers having a carboxylic acid group(s), such as 11 -(meth)acryloyloxyundecane-1, 1 -dicarboxylic acid, and 4-(meth)acryloyloxyethoxycarbonylphthalic acid.

The surface treatment method is not particularly limited, and known surface treatment methods may be employed.

There is no particular limitation to the amount of surface treatment agent used. For example, the surface treatment agent may be used in 0.1 parts or more by mass relative to 100 parts by mass of the filler before surface treatment. The amount of surface treatment agent is preferably 0.5 parts or more by mass, more preferably 1 part or more by mass, even more preferably 2 parts or more by mass, and may be 5 parts or more by mass, 10 parts or more by mass, or 20 parts or more by mass. The surface treatment agent is used in preferably 50 parts or less by mass, more preferably 45 parts or less by mass relative to 100 parts by mass of the filler before surface treatment. The amount of surface treatment agent may be 40 parts or less by mass, or 30 parts or less by mass. The mechanical strength of the cured product obtained can further improve when the amount of surface treatment agent is at or above these lower limits. With the amount of surface treatment agent at or below the foregoing upper limits, it is possible to reduce a decrease in the mechanical strength of the cured product due to the excess surface treatment agent.

The content of the filler (C) in a dental curable composition of the present invention is not particularly limited (the content of filler (C) after surface treatment when it is surface-treated). However, in view of considerations such as the handling properties of the dental curable composition and the mechanical strength of the cured product obtained, the content of the filler (C) in a dental curable composition of the present invention is preferably 10 mass% or more, more preferably 30 mass% or more, even more preferably 50 mass% or more, particularly preferably 65 mass% or more in 100 mass% of the dental curable composition. The content of filler (C) is preferably 97 mass% or less, more preferably 96 mass% or less, even more preferably 95 mass% or less, particularly preferably 90 mass% or less. When the content of filler (C) is at or above these lower limits, it improves the mechanical strength of the cured product, and more effectively reduces stickiness and stringiness in the dental curable composition, improving its handling properties. With the content of filler (C) at or below the forgoing upper limits, it is possible to prevent the dental curable composition from becoming excessively hard, leading to improved handling properties.

The filler (C) of the present invention preferably comprises a filler (C1) that has an average particle diameter of 0.001 $\mu$m or more and less than 0.1 $\mu$m, and that contains at least one metal (M) selected from the group consisting of aluminum, titanium, strontium, zirconium, barium, lanthanum, and ytterbium (hereinafter, also referred to simply as "filler (C1)"). The average particle diameter of filler (C1) is more preferably 0.002 $\mu$m or more and 0.08 $\mu$m or less, even more preferably 0.003 $\mu$m or more and 0.07 $\mu$m or less, particularly preferably 0.005 $\mu$m or more and 0.06 $\mu$m or less. With the average particle diameter of filler (C1) falling within these lower limit ranges, it is possible to obtain a dental curable composition having low viscosity with smooth handling properties. The effect of filler (C) on the transparency of the dental curable composition becomes smaller when the average particle diameter of filler (C) falls within the foregoing upper limit ranges. This can result in a dental curable composition with high aesthetic quality and a high depth of cure.

The average particle diameter of filler (C) can be determined by a laser diffraction scattering method or electron microscopy of particles. As an example of a laser diffraction scattering method, the average particle diameter can be measured by volume with a laser diffraction particle size analyzer (e.g., SALD-2300 manufactured by Shimadzu Corporation), using ethanol or a 0.2% sodium hexametaphosphate aqueous solution as dispersion medium. A laser diffraction scattering method is particularly convenient for the measurement of particles having a particle size of 0.1 $\mu$m or more. Here, 0.1 $\mu$m is a measured value by a laser diffraction scattering method.

For electron microscopy, a scanning electron microscope (e.g., SU3500, SU3800, or S-4000 manufactured by Hitachi High-Technologies Corporation) can be used.

As a specific example of electron microscopy, particles may be photographed with an electron microscope, and the size of particles (at least 100 or 200 particles, for example, 500 particles) observed in a unit field of the micrograph may be measured using image-analyzing particle size distribution measurement software (Mac-View manufactured by Mountech Co., Ltd.). The measurement may employ image processing software when the scanning electron microscope includes image processing software.

Here, the particle diameter is determined as the arithmetic mean value of the maximum and minimum lengths of particles, and the average particle diameter is calculated from the number of particles and the particle diameter.

In this specification, the average particle diameter of filler (C) means the average particle diameter before surface treatment when the filler (C) is surface-treated.

The filler (C1) is not particularly limited, as long as it is an inorganic fine particle containing a metal (M). The content of the metal (M) in the filler (C1) is preferably 1 mass% or more, more preferably 3 mass% or more, even more preferably 5 mass% or more, and may be 10 mass% or more, or 20 mass% or more. The content of the metal (M) in the filler (C1) is preferably 90 mass% or less, more preferably 85 mass% or less, even more preferably 80 mass% or less, and may be 70 mass% or less, or 60 mass% or less.

When the content of the metal (M) in the filler (C1) falls within ranges, it can result in a filler (C1) with a refractive index similar to that of polymerizable monomer (A) typically used for dental curable compositions, and that of a cured product (P) resulting from the polymerization of polymerizable monomer (A). This diminishes the effect of filler (C1) on the transparency of the dental curable composition, both before and after curing, leading to a dental curable composition with high aesthetic quality and a high depth of cure. When the content of the metal (M) in the filler (C1) is at or above the foregoing lower limits, it is possible to obtain a dental curable composition with high X-ray contrast. The content of the metal (M) in the filler (C1) can be measured using the method described in the EXAMPLES section below.

In this specification, the refractive index of cured product (P) is as described in the EXAMPLES section below.

Preferably, the refractive index of filler (C1) at 25°C satisfies the following formula [I-1].

$$0 \leq nC1\text{-}nA \leq 0.2 \qquad\qquad [\text{I-1}]$$

where nA and nC1 represent the refractive indices of polymerizable monomer (A) and filler (C1), respectively, at 25°C.

In formula [1-1], nC1-nA is more preferably 0.17 or less, even more preferably 0.14 or less.

With the refractive index of filler (C1) satisfying the formula above, it is possible to provide a dental curable composition having a high depth of cure. While the exact mechanism for this is not entirely clear, the following speculation has been made.

In general, when considering a composition where filler (C) and polymerizable monomer (A) are dispersed, if the average particle diameter of the filler (C) is 0.1 μm or more, a greater difference between the refractive indices of the filler (C) and polymerizable monomer (A) results in increased refraction and scattering of incident light at their interface, leading to a decrease in the transparency of the composition. This effect is less pronounced when the particle diameter of filler (C) is less than 0.1 μm. In the case of filler (C1), the small particle size of filler (C1) means that the refractive index difference between filler (C) and polymerizable monomer (A) has a small impact on the transparency of the composition. Upon curing this composition, the refractive index of the polymerizable monomer (A) typically increases by approximately 0.02 to 0.04 through polymerization. However, because of the small particle size of filler (C1), the filler (C1) has a small impact on the transparency of the cured product. By incorporating the filler (C-1) in filler (C), it is therefore possible to reduce changes in transparency before and after polymerization compared to when the filler (C1) is absent. This improves the transparency of the composition before and after polymerization, and can increase the depth of cure through exposure to light, depending on the combination of fillers (C).

When the refractive index of the filler (C1) additionally satisfies the formula above-meaning that it exceeds the refractive index of polymerizable monomer (A) to a certain extent-dispersing the filler (C1) in polymerizable monomer (A) increases the refractive index of the composition while having a small impact on its transparency. The filler (C1) acts similarly to an additive that increases the refractive index of polymerizable monomer (A).

The refractive index of the polymerizable monomer (A) with the filler (C1) dispersed therein is often comparable to or even higher than the refractive index of other fillers (C) described later. This increases the transparency of the dental curable composition, allowing light to penetrate more deeply and achieve a greater depth of cure. Conversely, as the dental curable composition becomes cured, the refractive index difference between the filler (C) and the cured product (P) of polymerizable monomer (A) increases, leading to a transparency decrease in the cured product of the dental curable composition, thereby allowing for a reduction in the amount of colorant (D) (described later), particularly a white pigment. This can result in a dental curable composition with a high depth of cure and superior color compatibility.

The refractive indices of filler (C1) and polymerizable monomer (A) can be measured using the method described in the EXAMPLES section below.

The content of filler (C1) is preferably 0.1 mass% or more, more preferably 0.5 mass% or more, even more preferably 1 mass% or more in 100 mass% of a dental curable composition of the present invention. The content of filler (C1) is preferably 30 mass% or less, more preferably 20 mass% or less, even more preferably 10 mass% or less. When the content of filler (C1) is within these lower limit ranges, it enhances the mechanical strength of the cured product of the dental curable composition, and allows the filler (C1) to exhibit its effect described above. With the content of filler (C1) falling within the foregoing upper limit ranges, it is possible to obtain a dental curable composition with low viscosity and smooth handling properties.

Preferably, the filler (C) of the present invention comprises a filler (C2) that has an average particle diameter of 1 μm or more and 50 μm or less, and that contains 5 mass% or more of at least one metal (M) selected from the group consisting of aluminum, titanium, strontium, zirconium, barium, lanthanum, and ytterbium (hereinafter, also referred to simply as "filler (C2)").

The average particle diameter of filler (C2) is more preferably 1 μm or more and 40 μm or less, even more preferably 3 μm or more and 30 μm or less. When the average particle diameter of filler (C2) is within these lower limit ranges, the resulting dental curable composition can have reduced stickiness and good handling properties. With the average particle diameter of filler (C2) falling within the foregoing upper limit ranges, it is possible to obtain a dental curable composition with reduced roughness and good handling properties.

The content of the metal (M) in the filler (C2) is preferably 5 mass% or more, more preferably 7 mass% or more, even more preferably 10 mass% or more. The content of the metal (M) in the filler (C2) is preferably 90 mass% or less, more preferably 85 mass% or less, even more preferably 80 mass% or less. When the content of the metal (M) in the filler (C2) falls within these ranges, it can result in a filler (C2) with a refractive index similar to that of polymerizable monomer (A) typically used for dental curable compositions. This increases transparency both before and after curing, leading to a dental curable composition with high aesthetic quality and a high depth of cure. When the content of the metal (M) in the filler (C2) is at or above the foregoing lower limits, it is possible to obtain a dental curable composition with high X-ray contrast. The content of the metal (M) in the filler (C2) can be measured using the method described in the EXAMPLES section below.

Preferably, the refractive index of filler (C2) at 25°C satisfies the following formula [I-2].

$$-0.03 \leq nC2-nA \leq 0.03 \qquad [I-2]$$

where nA and nC2 represent the refractive indices of polymerizable monomer (A) and filler (C2), respectively, at 25°C.

In formula [I-2], nC2-nA is more preferably -0.02 or more, even more preferably -0.015 or more. In formula [I-2], nC2-nA is more preferably 0.02 or less, even more preferably 0.015 or less.

With the refractive index of filler (C2) satisfying the foregoing formula, the polymerizable monomer (A) shows an increased refractive index upon curing, as noted above. While this provides high transparency in the dental curable composition before curing, it leads to a slight reduction in the transparency of the dental curable composition after curing. This enables a decrease in the content of the colorant (D) described below, allowing the dental curable composition to maintain high transparency before and after curing. As a result, a dental curable composition can be obtained that has a high depth of cure. The refractive indices of the filler (C2) and polymerizable monomer (A) can be measured using the method described in the EXAMPLES section below.

The content of filler (C2) is preferably 1 to 90 mass%, more preferably 5 to 80 mass%, even more preferably 10 to 70 mass% in 100 mass% of a dental curable composition of the present invention. When the content of filler (C2) is at or above these lower limits, the mechanical strength improves, and a dental curable composition can be obtained that exhibits good handling properties with no stickiness. When the content of filler (C2) is at or below the foregoing upper limits, the mechanical strength improves, and a dental curable composition can be obtained that exhibits good handling properties with no roughness.

Preferably, the filler (C2) is an inorganic agglomerated particle (C2-1) formed by agglomeration of inorganic primary particles (x), or an organic-inorganic composite filler (C2-2) containing inorganic primary particles (x). The inorganic primary particles (x) have an average particle diameter of preferably 0.001 $\mu$m or more and less than 1 $\mu$m.

The average particle diameter of inorganic primary particles (x) is more preferably 0.005 $\mu$m or more and 0.8 $\mu$m or less, even more preferably 0.01 $\mu$m or more and 0.5 $\mu$m or less.

When the average particle diameter of inorganic primary particles (x) is at or above these lower limits, the mechanical strength improves in the cured product of the dental curable composition. When the average particle diameter of inorganic primary particles (x) is at or below the foregoing upper limits, the resulting cured product of dental curable composition exhibits improved polishability and gloss retention. Additionally, this can result in a filling restoration with high color compatibility, making it easier to achieve glossiness comparable to that of natural teeth. The average particle diameter of inorganic primary particles (x) can be determined by electron microscopy.

In the present invention, the inorganic agglomerated particle (C2-1) has a form of an agglomerated particle formed by agglomeration of inorganic primary particles (x). Commercially available inorganic fillers typically exist in the form of aggregates. The cohesion of commercially available inorganic fillers is so weak that these fillers break into the particle size indicated by the manufacturer when 10 mg of its powder is added and ultrasonically dispersed at 40 W and 39 KHz for 30 minutes in 300 mL of a dispersion medium such as water, 5 mass% or less of a surfactant (e.g., sodium hexametaphosphate) in water, or ethanol. In contrast, the inorganic agglomerated particle (C2-1) of the present invention is strongly held together, and becomes hardly dispersed even under these conditions.

In a preferred method of preparing a strong agglomerate of particles from an aggregate of commercially available inorganic fillers, the inorganic filler is heated to a temperature range just below the temperature that melts the inorganic filler so that the adjoining inorganic filler particles, under the applied heat, lightly fuse together and increase cohesion. Here, the inorganic filler may have a form of an aggregate before heating, in order to control the shape of the agglomerated particle. An aggregate can be formed, for example, by applying pressure to the inorganic filler placed in a suitable container, or by dispersing the inorganic filler in a solvent, and removing the solvent using a method such as spray drying.

In another preferred method of preparing the inorganic agglomerated particle (C2-1) strongly held together by inorganic filler particles, a sol such as a silica sol, an alumina sol, a titania sol, or a zirconia sol prepared by a wet method is dried using a method such as freeze drying or spray drying, and optionally subjected to a heat treatment. In this way, the inorganic agglomerated particle (C2-1) can be obtained with ease that is strongly held together by particles.

Specific examples of the sols include fine spherical silica particles (Seahostar® manufactured by Nippon Shokubai Co., Ltd. under this trade name; e.g., KE series, a surface-treated type), a silica organosol (OSCAL® manufactured by JGC C & C under this trade name), a titania sol (QUEEN TITANIC series manufactured by Nissan Chemical Corporation under this trade name), a silica sol (SNOWTEX® manufactured by Nissan Chemical Corporation under this trade name), an alumina sol (Aluminasol-100, Aluminasol-200, Aluminasol-520 manufactured by Nissan Chemical Corporation under these trade names), and a zirconia sol (NanoUse® ZR series manufactured by Nissan Chemical Corporation under this trade name). The shape of the inorganic agglomerated particle (C2-1) is not particularly limited, and may be appropriately selected for use.

The heat treatment conditions in the method of production of inorganic agglomerated particle (C2-1) cannot be generalized as a rule because the optimum conditions (temperature, time) depend on factors such as the composition of the inorganic primary particles (x). For many compositions, however, the preferred heat treatment temperature (firing temperature) ranges from 500 to 1,200°C. An overly low heat treatment temperature tends to cause a decrease of mechanical strength in the cured product of dental composition finally obtained. An overly high heat treatment temperature causes excessive fusing between inorganic primary particles (x), and this tends to impair the polishability and gloss retention of the final cured product of dental composition.

More detailed processing conditions for the heat treatment can be determined by, for example, choosing conditions with which no crystalline structure can be confirmed in a powder X-ray diffraction analysis of inorganic agglomerated particles (C2-1) produced as secondary particles (agglomerated particles) under several firing conditions in the foregoing heat-treatment temperature ranges.

As another example, dental compositions may be produced from inorganic agglomerated particles (C2-1) produced in the manner described above, and the heat treatment conditions may be decided after measuring properties such as the flexural strength of cured products formed from these dental compositions, or gloss on polished surfaces of the cured products. In many cases, an insufficient heat treatment often leads to a cured product with insufficient flexural strength. When overly heat treated, the resultant cured product tends to have a low gloss on polished surfaces, in addition to showing an unnaturally opaque appearance. This is probably because an over heat treatment causes crystallization to occur in parts of the constituent components, increasing the refractive index of the inorganic agglomerated particle (C2-1), and producing an unnaturally white color, different from the whiteness of natural teeth, in a cured product formed by the dental composition using such an inorganic agglomerated particle (C2-1). An over heat treatment also increases the hardness of inorganic agglomerated particle (C2-1), and this appears to impair polishability by making it difficult to grind a cured product formed by the dental composition.

In the present invention, the inorganic agglomerated particle (C2-1) has a specific surface area of preferably 10 $m^2$/g or more, preferably 15 $m^2$/g or more, more preferably 18 $m^2$/g or more, even more preferably 20 $m^2$/g or more. The inorganic agglomerated particle (C2-1) has a specific surface area of 300 $m^2$/g or less, preferably 250 $m^2$/g or less, more preferably 200 $m^2$/g or less, even more preferably 190 $m^2$/g or less. The specific surface area of inorganic agglomerated particle (C2-1) may be 170 $m^2$/g or less, or 150 $m^2$/g or less.

When the specific surface area of inorganic agglomerated particle (C2-1) is at or above these lower limits, the cured product of dental curable composition obtained can have improved polishability, and improved color compatibility. When the specific surface area of inorganic agglomerated particle (C2-1) is at or below the foregoing upper limits, it is possible to increase the content of inorganic agglomerated particle (C2-1), and increase the mechanical strength of the cured product obtained.

The specific surface area of inorganic agglomerated particle (C2-1) means a specific surface area of the agglomerated particle (secondary particle).

The specific surface area of inorganic agglomerated particle (C2-1) can be determined by the BET method. Specifically, the specific surface area of inorganic agglomerated particle (C2-1) can be measured using, for example, a specific surface area measurement device (e.g., BELSORP-mini series manufactured by MicrotracBEL Corp.). For precision, the specific surface area is determined through the evaluation of specific surface area analyzed by the multi-point BET method from the adsorption isotherm measured in high-accuracy mode.

More specifically, the specific surface area of inorganic agglomerated particle (C2-1) can be measured under the following measurement conditions.

Using a specific surface area measurement instrument (BELSORP-mini II manufactured by MicrotracBEL Corp.), the sample is degassed in vacuum at 100°C for 2 hours. The measurement can then be carried out with nitrogen as the adsorbate gas, at a temperature of 77 K, using the multi-point BET method (high-precision mode) with 5 points on the adsorption isotherm where the ratio (P/P0) of adsorption equilibrium pressure P (kPa) to saturation vapor pressure P0 (kPa) is 0.05 to 0.3.

In the present invention, the organic-inorganic composite filler (C2-2) refers to a filler comprising the inorganic primary particles (x) and a polymer of polymerizable monomer (A').

The method for producing the organic-inorganic composite filler (C2-2) is not particularly limited in the present invention. For example, a polymerizable monomer (A') and a known polymerization initiator may be added into the inorganic primary particles (x) to form a paste in advance. The paste can then undergo polymerization through solution polymerization, suspension polymerization, emulsion polymerization, or bulk polymerization, followed by pulverization.

The content of the inorganic primary particles (x) in the organic-inorganic composite filler (C2-2) is preferably 10 parts or more by mass, more preferably 20 parts or more by mass, even more preferably 30 parts or more by mass, particularly preferably 40 parts or more by mass relative to total 100 parts by mass of the inorganic primary particles (x) and polymerizable monomer (A') in the organic-inorganic composite filler (C2-2).

When the content of inorganic primary particles (x) falls within these lower limit ranges, the mechanical strength improves in the cured product of the dental curable composition. The upper limits are not particularly limited, and are preferably 99 parts or less by mass, more preferably 95 parts or less by mass to avoid a viscosity increase in the paste due to production.

Preferred for use as the polymerizable monomer (A') are the polymerizable monomers listed as examples of polymerizable monomer (A) usable in a dental curable composition of the present invention. In such polymerizable monomers, the absolute value of the refractive index difference between the polymerizable monomer (A') after curing and the inorganic primary particles (x) in the organic-inorganic composite filler (C2-2) is preferably 0.30 or less, more preferably 0.20 or less. When the refractive index difference is at or below these upper limits, the transparency of the organic-inorganic composite filler (C2-2) itself can improve, leading to a dental curable composition with high aesthetic quality.

Preferably, the filler (C) of the present invention comprises a light-diffusing filler (C3) that has an average particle diameter of 1 μm or more and 50 μm or less, and that contains 0 mass% or more and less than 5 mass% of at least one metal (M) selected from the group consisting of aluminum, titanium, strontium, zirconium, barium, lanthanum, and ytterbium (hereinafter, also referred to simply as "filler (C3)").

The average particle diameter of filler (C3) is more preferably 1 μm or more and 40 μm or less, even more preferably 1 μm or more and 30 μm or less. With the average particle diameter of filler (C3) falling within these ranges, it is possible to impart appropriate light diffusing properties to the cured product of the dental curable composition, making it easier to obtain a highly aesthetic dental curable composition with a single formulation in the treatment of molars, particularly Class II cavities in molars that include proximal surfaces.

Furthermore, with the average particle diameter of filler (C3) falling within the foregoing ranges, superior form retention can be imparted to the dental curable composition, resulting in a dental curable composition that is easy to work with, particularly in the treatment of Class II cavities in molars, which involves imparting complex shapes including proximal surfaces.

A certain preferred embodiment is, for example, a dental curable composition in which the filler (C) in any of the embodiments above comprises the filler (C2) and/or filler (C3). Through different combinations of fillers (C) and different combinations of parameters such content, particle size, refractive index difference, and comparative diffusivity, it is possible to more easily achieve favorable color compatibility even in molars with Class II cavities of about 4 mm depth involving proximal surfaces.

Another certain preferred embodiment is, for example, a dental curable composition in which the filler (C) in any of the embodiments above comprises the filler (C1). Through different combinations of fillers (C) and different combinations of parameters such content, particle size, refractive index difference, and comparative diffusivity, it is possible to more easily achieve favorable color compatibility even in molars with Class II cavities of about 4 mm depth involving proximal surfaces.

Yet another certain preferred embodiment is, for example, a dental curable composition in which the filler (C) in any of the embodiments above comprises the filler (C1), filler (C2), and filler (C3). This combination of fillers (C) increases the packing ratio of fillers, enhancing the interactions between the fillers, and helping the paste retain its shape under gravity.

Favorable form retention can be obtained in a particularly preferred embodiment where the dental curable composition comprises the filler (C3).

The filler (C3) is not particularly limited, as long as it is a filler possessing light diffusing properties. However, the content of the metal (M) in the filler (C3) is preferably less than 5 mass%, more preferably less than 3 mass%, even more preferably less than 1 mass%, or the metal (M) may be absent (0 mass%).

When the content of the metal (M) in the filler (C3) falls within these upper limit ranges, the filler (C3) can have a lower refractive index than the cured product (P) obtained through polymerization of polymerizable monomer (A) typically used for dental curable compositions.

Employing the filler (C3) with a refractive index lower than that of the cured product (P) allows adequate light diffusing properties to be imparted to the cured product of the dental curable composition, leading to a dental curable composition with high aesthetic quality. The content of the metal (M) in the filler (C3) can be measured using the method described in the EXAMPLES section below.

Preferably, the refractive index of filler (C3) at 25°C satisfies the following formula [I-3].

$$-0.2 \leq nC3\text{-}nA \leq 0 \qquad\qquad [\text{I-3}]$$

where nA and nC3 represent the refractive indices of polymerizable monomer (A) and filler (C3), respectively, at 25°C.

In formula [I-3], nC3-nA is more preferably -0.15 or more, even more preferably -0.1 or more.

With the refractive index of filler (C3) satisfying the foregoing formula, the polymerizable monomer (A) shows an increased refractive index upon curing, creating a refractive index difference between the filler (C3) and the cured product (P) obtained from the polymerizable monomer trough polymerization. By allowing incident light to scatter at the boundary between the filler (C3) and cured product (P), it is possible to provide a dental curable composition possessing adequate light diffusing properties. The refractive indices of the filler (C3) and polymerizable monomer (A) can be measured using the method described in the EXAMPLES section below.

In view of helping provide more favorable color compatibility with a wide range of natural tooth shades with a dental curable composition using a single formulation, the filler (C) of the present invention preferably comprises a light-diffusing inorganic agglomerated particle (C3-1) or a light-diffusing organic-inorganic composite filler (C3-2). Alternatively, the filler (C) may comprise both of these fillers.

Another certain preferred embodiment is, for example, a dental curable composition in which the filler (C) comprises the filler (C1), filler (C2), light-diffusing inorganic agglomerated particle (C3-1), and light-diffusing organic-inorganic composite filler (C3-2). By combining these fillers (C), the light passing through the cured product becomes adequately diffused, which helps to blur the boundary between the natural tooth and the portions filled with the dental curable composition. It also reduces a dark dull impression in the shade of the filled portion even in deeper cavities. Additionally, the shade of the cavity floor of natural teeth can more easily manifest in the filled portion when the refractive indices are adjusted to satisfy the predetermined formulae ([I-1] to [I-3]).

The content of filler (C3) is preferably 0.1 to 30 mass%, more preferably 0.5 to 25 mass%, even more preferably 1 to 20 mass% in 100 mass% of a dental curable composition of the present invention. The content of filler (C3) can be appropriately selected from these ranges, and may be 15 mass% or less, or 10 mass% or less. When the content of filler (C3) falls within these ranges, a dental curable composition with adequate light diffusing properties can be obtained, and the color compatibility improves.

Preferably, the filler (C3) is a light-diffusing inorganic agglomerated particle (C3-1) formed by agglomeration of inorganic primary particles (x), or a light-diffusing organic-inorganic composite filler (C3-2) containing inorganic primary particles (x). The inorganic primary particles (x) have an average particle diameter of preferably 0.001 $\mu$m or more and less than 1 $\mu$m.

The average particle diameter of inorganic primary particles (x) is more preferably 0.005 $\mu$m or more and 0.8 $\mu$m or less, even more preferably 0.01 $\mu$m or more and 0.5 $\mu$m or less. When the average particle diameter of inorganic primary particles (x) is at or above these lower limits, the mechanical strength improves in the cured product of the dental curable composition.

When the average particle diameter of inorganic primary particles (x) falls within the foregoing upper limit ranges, the resulting cured product of dental curable composition exhibits improved polishability and gloss retention. Additionally, this can result in a filling restoration with high color compatibility, making it easier to achieve glossiness comparable to that of natural teeth. The average particle diameter of inorganic primary particles (x) can be determined by electron microscopy.

In the present invention, the light-diffusing inorganic agglomerated particle (C3-1) has a form of an agglomerated particle formed by agglomeration of inorganic primary particles (x). Commercially available inorganic fillers typically exist in the form of aggregates. The cohesion of commercially available inorganic fillers is so weak that these fillers break into the particle size indicated by the manufacturer when 10 mg of its powder is added and ultrasonically dispersed at 40 W and 39 KHz for 30 minutes in 300 mL of a dispersion medium such as water, 5 mass% or less of a surfactant (e.g., sodium hexametaphosphate) in water, or ethanol. In contrast, the light-diffusing inorganic agglomerated particle (C3-1) of the present invention is strongly held together, and becomes hardly dispersed even under these conditions.

The light-diffusing inorganic agglomerated particle (C3-1) in the present invention can be produced using the same method employed for the inorganic agglomerated particle (C2-1).

Any of the raw materials listed for the inorganic agglomerated particle (C2-1) can be used for inorganic primary particles (x) without any limitation. However, in order to control the refractive index, particularly preferred are various glass powders primarily made of silica, such as fused silica, quartz, soda-lime-silica glass, E glass, C glass, borosilicate glass (PYREX® glass), and fumed silica. These may be used alone, or two or more thereof may be used in combination.

In the present invention, the light-diffusing inorganic agglomerated particle (C3-1) has a specific surface area of preferably 10 m²/g or more, preferably 15 m²/g or more, more preferably 18 m²/g or more, even more preferably 20 m²/g or more. The light-diffusing inorganic agglomerated particle (C3-1) has a specific surface area of 300 m²/g or less, preferably 250 m²/g or less, more preferably 200 m²/g or less, even more preferably 190 m²/g or less. The specific surface area of light-diffusing inorganic agglomerated particle (C3-1) may be 170 m²/g or less, or 150 m²/g or less.

When the specific surface area of light-diffusing inorganic agglomerated particle (C3-1) is at or above these lower limits, the cured product of dental curable composition obtained can have improved polishability, and improved color compatibility.

When the specific surface area of light-diffusing inorganic agglomerated particle (C3-1) is at or below the foregoing upper limits, it is possible to increase the content of light-diffusing inorganic agglomerated particle (C3-1), enhancing the mechanical strength of the cured product obtained. The specific surface area of light-diffusing inorganic agglomerated particle (C3-1) means a specific surface area of the agglomerated particle (secondary particle).

The specific surface area of light-diffusing inorganic agglomerated particle (C3-1) can be measured using the same method employed for the inorganic agglomerated particle (C2-1).

The light-diffusing organic-inorganic composite filler (C3-2) in the present invention refers to a filler comprising the inorganic primary particles (x) and a polymer of polymerizable monomer (A').

The method for producing the light-diffusing organic-inorganic composite filler (C3-2) is not particularly limited in the present invention. The light-diffusing organic-inorganic composite filler (C3-2) can be produced using the same method employed for the organic-inorganic composite filler (C2-2).

Any of the raw materials listed for the inorganic agglomerated particle (C2-1) can be used for inorganic primary particles (x) without any limitation. However, in order to control the refractive index, particularly preferred are various glass powders primarily made of silica, such as fused silica, quartz, soda-lime-silica glass, E glass, C glass, borosilicate glass (PYREX® glass), and fumed silica. These may be used alone, or two or more thereof may be used in combination.

The content of the inorganic primary particles (x) in the light-diffusing organic-inorganic composite filler (C3-2) is preferably 10 parts or more by mass, more preferably 20 parts or more by mass, even more preferably 30 parts or more by mass, particularly preferably 40 parts or more by mass relative to total 100 parts by mass of the inorganic primary particles (x) and polymerizable monomer (A') in the light-diffusing organic-inorganic composite filler (C3-2). When the content of inorganic primary particles (x) is at or above these lower limits, the mechanical strength improves in the cured product of the dental curable composition. The upper limits are not particularly limited, and are preferably 99 parts or less by mass, more preferably 95 parts or less by mass to avoid a viscosity increase in the paste due to production.

Preferred for use as the polymerizable monomer (A') are the polymerizable monomers listed as examples of polymerizable monomer (A) usable in a dental curable composition of the present invention. In such polymerizable monomers, the absolute value of the refractive index difference between the polymerizable monomer (A') after curing and the inorganic primary particles (x) in the light-diffusing organic-inorganic composite filler (C3-2) is preferably 0.30 or less, more preferably 0.20 or less. When the refractive index difference is at or below these upper limits, the transparency of the light-diffusing organic-inorganic composite filler (C3-2) itself can improve, leading to a dental curable composition with high aesthetic quality.

Preferably, the filler (C) of the present invention comprises a filler (C4) having an average particle diameter of 0.1 $\mu$m or more and less than 1 $\mu$m (hereinafter, also referred to simply as "filler (C4)"). The average particle diameter of filler (C4) is more preferably 0.1 $\mu$m or more and 0.9 $\mu$m or less, even more preferably 0.1 $\mu$m or more and 0.8 $\mu$m or less. The average particle diameter of filler (C4) can be appropriately selected from the foregoing ranges, and may be 0.1 $\mu$m or more and 0.7 $\mu$m or less, 0.1 $\mu$m or more and 0.6 $\mu$m or less, or 0.1 $\mu$m or more and 0.5 $\mu$m or less. When the average particle diameter of filler (C4) falls within these lower limit ranges, the resulting dental curable composition can have good handling properties with reduced stickiness, and superior mechanical strength. When the average particle diameter of filler (C4) falls within the foregoing upper limit ranges, the resulting cured product of dental curable composition exhibits improved polishability and gloss retention. Additionally, this can result in a filling restoration with high color compatibility, making it easier to achieve glossiness comparable to that of natural teeth.

Preferably, the filler (C4) comprises at least one metal (M) selected from the group consisting of aluminum, titanium, strontium, zirconium, barium, lanthanum, and ytterbium. The content of the metal (M) in the filler (C4) is preferably 5 mass% or more, more preferably 7 mass% or more, even more preferably 10 mass% or more. The content of the metal (M) in the filler (C4) is preferably 90 mass% or less, more preferably 85 mass% or less, even more preferably 80 mass% or less. When the content of the metal (M) in the filler (C4) falls within these ranges, the filler (C4) can have a refractive index similar to that of polymerizable monomer (A) typically used for dental curable compositions. This increases transparency both before and after curing, leading to a dental curable composition with high aesthetic quality and a high depth of cure. When the content of the metal (M) in the filler (C4) is at or above the foregoing lower limits, it is possible to obtain a dental curable composition with high X-ray contrast. The content of the metal (M) in the filler (C4) can be measured using the method described in the EXAMPLES section below.

Preferably, the refractive index of filler (C4) at 25°C satisfies the following formula [I-4].

$$-0.035 \leq nC4\text{-}nA \leq 0.035 \qquad [\text{I-4}]$$

where nA and nC4 represent the refractive indices of polymerizable monomer (A) and filler (C4), respectively, at 25°C.

In formula [I-4], nC4-nA is more preferably -0.02 or more, even more preferably -0.015 or more. In formula [I-4], nC4-nA is more preferably 0.02 or less, even more preferably 0.015 or less.

With the refractive index of filler (C4) satisfying the foregoing formula, the polymerizable monomer (A) shows an increased refractive index upon curing, as noted above. While this provides high transparency in the dental curable composition before curing, it leads to a slight reduction in the transparency of the dental curable composition after curing. This enables a decrease in the content of the colorant (D) described below, allowing the dental curable composition to maintain high transparency before and after curing. As a result, a dental curable composition can be obtained that has a high depth of cure. The refractive indices of the filler (C4) and polymerizable monomer (A) can be measured using the method described in the EXAMPLES section below.

A certain preferred embodiment is, for example, a dental curable composition in which the filler (C) in any of the embodiments above comprises at least one filler selected from the group consisting of the filler (C1), filler (C2), filler (C3), and filler (C4).

Another certain preferred embodiment is, for example, a dental curable composition in which the filler (C) in any of the embodiments above comprises at least two fillers selected from the group consisting of the filler (C1), filler (C2), filler (C3), and filler (C4).

A certain preferred embodiment is, for example, a dental curable composition in which the filler (C) in any of the embodiments above comprises the filler (C3) and filler (C4).

Through the combination of filler (C3) and filler (C4) and different combinations of parameters such content, particle size, refractive index difference, and comparative diffusivity, it is possible to more easily achieve favorable color compatibility even in molars with Class II cavities of about 4 mm depth involving proximal surfaces, enabling the dental curable composition to more easily exhibit favorable color compatibility with a wide range of natural tooth shades using a single formulation.

Another certain preferred embodiment is, for example, a dental curable composition in which the filler (C3) and filler (C4) in any of the embodiments above satisfy the following formulae [I-3] and [I-4], respectively.

$$-0.2 \leq nC3\text{-}nA \leq 0 \qquad [\text{I-3}]$$

$$-0.035 \leq nC4\text{-}nA \leq 0.035 \qquad [\text{I-4}]$$

In the formulae, the symbols have the same meanings as previously described.

Yet another certain preferred embodiment is, for example, a dental curable composition in which the filler (C) in any of the embodiments above comprises the filler (C1), filler (C2), filler (C3), and filler (C4).

Through the combination of these four fillers and different combinations of parameters such content, particle size, refractive index difference, and comparative diffusivity, it is possible to more easily achieve favorable color compatibility even in molars with Class II cavities of about 4 mm depth involving proximal surfaces.

Still another certain preferred embodiment is, for example, a dental curable composition in which the total content of the filler (C1), filler (C2), filler (C3), and filler (C4) in any of the embodiments above is 10 to 97 mass%.

Yet another certain preferred embodiment is, for example, a dental curable composition in which the filler (C1), filler (C2), filler (C3), and filler (C4) in any of the embodiments above satisfy the following formulae [I-1], [I-2], [I-3], and [I-4], respectively.

$$0 \leq nC1\text{-}nA \leq 0.2 \qquad [\text{I-1}]$$

$$-0.03 \leq nC2\text{-}nA \leq 0.03 \qquad [\text{I-2}]$$

$$-0.2 \leq nC3\text{-}nA \leq 0 \qquad [\text{I-3}]$$

$$-0.035 \leq nC4\text{-}nA \leq 0.035 \qquad [\text{I-4}]$$

In the formulae, the symbols have the same meanings as previously described.

The content of the filler (C4) is preferably 1 to 80 mass%, more preferably 5 to 70 mass%, even more preferably 10 to 60 mass% in 100 mass% of a dental curable composition of the present invention. The content of filler (C4) may be 15 to 50 mass%, or 20 to 40 mass%. When the content of filler (C4) is at or above these lower limits, it leads to a dental curable composition with superior mechanical strength. When the content of filler (C4) is at or below the foregoing upper limits, it leads to a dental curable composition having good handling properties with reduced stickiness.

The filler (C) in the present invention may additionally comprise a filler (C5) that has an average particle diameter of 0.001 to 0.1 $\mu$m, and that does not contain any of the metals (M) aluminum, titanium, strontium, zirconium, barium, lanthanum, and ytterbium (hereinafter, also referred to simply as "additional filler (C5)"). The additional filler (C5) may be inorganic fine particles. Preferably, the additional filler (C5) contains silica (with a silica content of 5 mass% or more, preferably 10 mass% or more).

The content of the additional filler (C5) in a dental curable composition of the present invention is not particularly limited. Alternatively, the additional filler (C5) may be absent. A dental curable composition comprising the additional filler (C5) is smooth and easy to handle, and excels in mechanical strength. However, because the additional filler (C5) tends to decrease the transparency of the cured product, the content of the additional filler (C5) in a dental curable composition of the present invention is preferably 10 mass% or less in 100 mass% of the dental curable composition. The content of additional filler (C5) may be 5 mass% or less, 3 mass% or less, or less than 1 mass%. Alternatively, the additional filler (C5) may be absent.

The filler (C) in a dental curable composition of the present invention may be a filler with a number-based particle size distribution where 90% or more of the particles are within $\pm5\%$ of the average primary particle diameter. However, the filler (C) is not necessarily required to have such a monodisperse distribution.

[Colorant (D)]

A dental curable composition of the present invention may comprise a colorant (D). There are not specific restrictions on the type and other attributes of colorant (D). Any inorganic pigments and/or organic pigments may be used according to the intended shade of the dental curable composition, without any limitation. For each colorant, the colorant content is less than 0.1 mass% in 100 mass% of the dental curable composition. The shape of color particles is not particularly limited, and may be any of various shapes, including, for example, spherical, stylus, plate-like, crushed, and scale-like shapes.

Specific examples of inorganic pigments include chromates such as chrome yellow, zinc yellow, and barium yellow; ferrocyanides such as iron blue; sulfides such as silver vermilion, cadmium yellow, zinc sulfide, and cadmium red; sulfates such as barium sulfate, zinc sulfate, and strontium sulfate; oxides such as zinc white, antimony white, titanium white (titanium oxide), red iron oxide, iron black, and chromium oxide; hydroxides such as aluminum hydroxide; silicates such as calcium silicate, and ultramarine; and carbons such as carbon black, and graphite. Specific examples of organic pigments include nitroso pigments such as naphthol green B, and naphthol green Y; nitro pigments such as naphthol yellow S, and xylene fast yellow 2G; insoluble azo pigments such as toluidine red, brilliant fast scarlet, Hansa yellow, and pigment yellow 14; poorly soluble azo pigments such as lithol red, lake red C, and lake red D; soluble azo pigments such as brilliant carmine 6B, toluidine red F5R, pigment scarlet 3B, and bordeaux 10B; phthalocyanine pigments such as phthalocyanine blue, phthalocyanine green, and sky blue; basic dye pigments such as rhodamine lake, malachite green lake, and methyl violet lake; and acidic dye pigments such as peacock blue lake, eosin lake, quinoline yellow lake, and aluminum lake. The colorant (D) may be used alone, or two or more thereof may be used in combination. Preferred among these colorants (D) are inorganic pigments, which are superior to organic pigments in terms of properties such as heat resistance and lightfastness. Particularly preferred are, for example, titanium white, red iron oxide, iron black, and yellow ferrous oxide.

Preferred for use as inorganic pigments are those with a refractive index higher than 2.00 at 25°C, distinct from the filler (C). The inorganic pigments may have a refractive index of 2.05 or more, or 2.10 or more at 25°C.

The content of the colorant (D) in a dental curable composition of the present invention is not particularly limited, as long as the present invention can exhibit its effects. However, in view of aesthetics, the content of colorant (D) is preferably 0.0005 parts or more by mass, more preferably 0.002 parts or more by mass, even more preferably 0.006 parts or more by mass, particularly preferably 0.01 parts or more by mass relative to 100 parts by mass of polymerizable monomer (A). When the content of colorant (D) is at or above these lower limits, it is possible to effectively reduce a dark dull impression in the filled portion. The content of colorant (D) is preferably 0.5 parts or less by mass, more preferably 0.3 parts or less by mass, even more preferably 0.2 parts or less by mass, particularly preferably 0.08 parts or less by mass. When the content of colorant (D) is at or below these upper limits, the shade of the cavity floor can effectively manifest in the filled portion.

The content of colorant (D) is preferably 0.0001 mass% or more, more preferably 0.0004 mass% or more, even more preferably 0.0012 parts or more by mass, particularly preferably 0.002 mass% or more relative to 100 mass% of the dental curable composition. The content of colorant (D) is preferably 0.1 mass% or less, more preferably 0.06 mass% or less, even more preferably 0.04 mass% or less.

[Polymer (E)]

A dental curable composition of the present invention may comprise a polymer (E). The polymer (E) may be a prepolymer or an oligomer. Preferred for use as polymer (E) are oligomers and polymers having radical polymerizable groups such as (meth)acrylic acid ester groups. For example, it is possible to use the polymerizable prepolymers described in JP S50-42696 A, the unsaturated urethane-based oligomers described in JP 2011-144121 A, the multiacrylate compounds described in JP 2006-510583 T, and the prepolymers described in WO2020/122192. The polymer (E) may be used alone, or two or more thereof may be used in combination.

The content of the polymer (E) in a dental curable composition of the present invention is not particularly limited. When polymer (E) is incorporated, it is possible to reduce the polymerization shrinkage stress or volumetric shrinkage that occurs while curing the dental curable composition, without compromising the mechanical properties of the cured product of dental curable composition. In view of reducing a decrease in the handling properties of the dental curable composition due to increased stickiness and viscosity, the content of the polymer (E) in a dental curable composition of the present invention is preferably 30 mass% or less, more preferably 10 mass% or less in 100 mass% of the dental curable composition. The content of polymer (E) may be 5 mass% or less, or 1 mass% or less. Alternatively, the polymer (E) may be absent.

In view of the mechanical strength of the cured product of dental curable composition obtained, it is preferable that the polymer (E) used in the present invention be compatible with the polymerizable monomer (A).

[Polymerization Accelerator (F)]

A dental curable composition of the present invention may additionally comprise a polymerization accelerator (F). Examples of the polymerization accelerator include amines, sulfinic acid and salts thereof, borate compounds, barbituric acid compounds, triazine compounds, copper compounds, tin compounds, vanadium compounds, halogen compounds, aldehydes, thiol compounds, sulfites, bisulfites, thiourea compounds, aryliodonium salt compounds, sulfonium salt compounds, and sulfonic acid ester compounds. The polymerization accelerator (F) may be used alone, or two or more thereof may be used in combination.

The amines can be classified into aliphatic amines and aromatic amines. Examples of the aliphatic amines include primary aliphatic amines such as n-butylamine, n-hexylamine, and n-octylamine; secondary aliphatic amines such as diisopropylamine, dibutylamine, and N-methylethanolamine; and tertiary aliphatic amines such as N-methyldiethanolamine, N-ethyldiethanolamine, N-n-butyldiethanolamine, N-lauryldiethanolamine, 2-(dimethylamino)ethyl methacrylate, N-methyldiethanolamine dimethacrylate, N-ethyldiethanolamine dimethacrylate, triethanolamine monomethacrylate, triethanolamine dimethacrylate, triethanolamine trimethacrylate, triethanolamine, trimethylamine, triethylamine, and tributylamine. In view of curability and storage stability of the dental curable composition, preferred are tertiary aliphatic amines, more preferably N-methyldiethanolamine and triethanolamine.

Examples of the aromatic amines include N,N-bis(2-hydroxyethyl)-3,5-dimethylaniline, N,N-bis(2-hydroxyethyl)-p-toluidine, N,N-bis(2-hydroxyethyl)-3,4-dimethylaniline, N,N-bis(2-hydroxyethyl)-4-ethylaniline, N,N-bis(2-hydroxyethyl)-4-isopropylaniline, N,N-bis(2-hydroxyethyl)-4-t-butylaniline, N,N-bis(2-hydroxyethyl)-3,5-di-isopropylaniline, N,N-bis(2-hydroxyethyl)-3,5-di-t-butylaniline, N,N-dimethylaniline, N,N-dimethyl-p-toluidine, N,N-dimethyl-m-toluidine, N,N-diethyl-p-toluidine, N,N-dimethyl-3,5-dimethylaniline, N,N-dimethyl-3,4-dimethylaniline, N,N-dimethyl-4-ethylaniline, N,N-dimethyl-4-isopropylaniline, N,N-dimethyl-4-t-butylaniline, N,N-dimethyl-3,5-di-t-butylaniline, ethyl 4-(N,N-dimethylamino)benzoate, methyl 4-(N,N-dimethylamino)benzoate, 2-butoxyethyl 4-(N,N-dimethylamino)benzoate, 2-(methacryloyloxy)ethyl 4-(N,N-dimethylamino)benzoate, 4-(N,N-dimethylamino)benzophenone, and butyl 4-(N,N-dimethylamino)benzoate. In view of the ability to impart superior curability to the dental curable composition, preferred for use is at least one selected from the group consisting of N,N-bis(2-hydroxyethyl)-p-toluidine, ethyl 4-(N,N-dimethylamino)benzoate, 2-butoxyethyl 4-(N,N-dimethylamino)benzoate, and 4-(N,N-dimethylamino)benzophenone.

Examples of the sulfinic acid and salts thereof include p-toluenesulfinic acid, sodium p-toluenesulfinate, potassium p-toluenesulfinate, lithium p-toluenesulfinate, calcium p-toluenesulfinate, benzenesulfinic acid, sodium benzenesulfinate, potassium benzenesulfinate, lithium benzenesulfinate, calcium benzenesulfinate, 2,4,6-trimethylbenzenesulfinic acid, sodium 2,4,6-trimethylbenzenesulfinate, potassium 2,4,6-trimethylbenzenesulfinate, lithium 2,4,6-trimethylbenzenesulfinate, calcium 2,4,6-trimethylbenzenesulfinate, 2,4,6-triethylbenzenesulfinic acid, sodium 2,4,6-triethylbenzenesulfinate, potassium 2,4,6-triethylbenzenesulfinate, lithium 2,4,6-triethylbenzenesulfinate, calcium 2,4,6-triethylbenzenesulfinate, 2,4,6-triisopropylbenzenesulfinic acid, sodium 2,4,6-triisopropylbenzenesulfinate, potassium 2,4,6-triisopropylbenzenesulfinate, lithium 2,4,6-triisopropylbenzenesulfinate, and calcium 2,4,6-triisopropylbenzenesulfinate. Preferred are sodium benzenesulfinate, sodium p-toluenesulfinate, and sodium 2,4,6-triisopropylbenzenesulfinate.

The borate compounds are preferably arylborate compounds. Examples of the arylborate compounds include borate compounds having one aryl group per molecule, borate compounds having two aryl groups per molecule, borate compounds having three aryl groups per molecule, and borate compounds having four aryl groups per molecule. In view of storage stability, preferred are borate compounds having three or four aryl groups per molecule.

Examples of the borate compounds having one aryl group per molecule include trialkylphenylboron, trialkyl(p-chlorophenyl)boron, trialkyl(p-fluorophenyl)boron, trialkyl[3,5-bis(trifluoromethyl)phenyl]boron, trialkyl[3,5-bis(1,1,1,3,3,3-hexafluoro-2-methoxy-2-propyl)phenyl]boron, trialkyl(p-nitrophenyl)boron, trialkyl(m-nitrophenyl)boron, trialkyl(p-butylphenyl)boron, trialkyl(m-butylphenyl)boron, trialkyl(p-butyloxyphenyl)boron, trialkyl(m-butyloxyphenyl)boron, trialkyl(p-octyloxyphenyl)boron, trialkyl(m-octyloxyphenyl)boron (the alkyl group is at least one selected from the group consisting of, for example, n-butyl, n-octyl, and n-dodecyl), and salts of these (e.g., sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts).

Examples of the borate compounds having three aryl groups per molecule include monoalkyl triphenylboron, monoalkyl tri(p-chlorophenyl)boron, monoalkyl tri(p-fluorophenyl)boron, monoalkyl tri[3,5-bis(trifluoromethyl)phenyl]boron, monoalkyl tri[3,5-bis(1,1,1,3,3,3-hexafluoro-2-methoxy-2-propyl)phenyl]boron, monoalkyl tri(p-nitrophenyl)boron, monoalkyl tri(m-nitrophenyl)boron, monoalkyl tri(p-butylphenyl)boron, monoalkyl tri(m-butylphenyl)boron, monoalkyl tri(p-butyloxyphenyl)boron, monoalkyl tri(m-butyloxyphenyl)boron, monoalkyl tri(p-octyloxyphenyl)boron, monoalkyl tri(m-octyloxyphenyl)boron (the alkyl group is one selected from, for example, n-butyl, n-octyl, and n-dodecyl), and salts of these (e.g., sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts).

Examples of the borate compounds having four aryl groups per molecule include tetraphenylboron, tetrakis(p-chlorophenyl)boron, tetrakis(p-fluorophenyl)boron, tetrakis[3,5-bis(trifluoromethyl)phenyl]boron, tetrakis[3,5-bis(1,1,1,3,3,3-hexafluoro-2-methoxy-2-propyl)phenyl]boron, tetrakis(p-nitrophenyl)boron, tetrakis(m-nitrophenyl)boron, tetrakis(p-butylphenyl)boron, tetrakis(m-butylphenyl)boron, tetrakis(p-butyloxyphenyl)boron, tetrakis(m-butyloxyphenyl)boron, tetrakis(p-octyloxyphenyl)boron, tetrakis(m-octyloxyphenyl)boron, (p-fluorophenyl)triphenylboron, [3,5-bis(trifluoromethyl)phenyl]triphenylboron, (p-nitrophenyl)triphenylboron, (m-butyloxyphenyl)triphenylboron, (p-butyloxyphenyl)triphenylboron, (m-octyloxyphenyl)triphenylboron, (p-octyloxyphenyl)triphenylboron, and salts of these (e.g., sodium salts, lithium salts, potassium salts, magnesium salts, tetrabutylammonium salts, tetramethylammonium salts, tetraethylammonium salts, methylpyridinium salts, ethylpyridinium salts, butylpyridinium salts, methylquinolinium salts, ethylquinolinium salts, and butylquinolinium salts).

Examples of the barbituric acid compounds include barbituric acid, 1,3-dimethylbarbituric acid, 1,3-diphenylbarbituric acid, 1,5-dimethylbarbituric acid, 5-butylbarbituric acid, 5-ethylbarbituric acid, 5-isopropylbarbituric acid, 5-cyclohexylbarbituric acid, 1,3,5-trimethylbarbituric acid, 1,3-dimethyl-5-ethylbarbituric acid, 1,3-dimethyl-5-n-butylbarbituric acid, 1,3-dimethyl-5-isobutylbarbituric acid, 1,3-dimethyl-5-cyclopentylbarbituric acid, 1,3-dimethyl-5-cyclohexylbarbituric acid, 1,3-dimethyl-5-phenylbarbituric acid, 1-cyclohexyl-1-ethylbarbituric acid, 1-benzyl-5-phenylbarbituric acid, 5-methylbarbituric acid, 5-propylbarbituric acid, 1,5-diethylbarbituric acid, 1-ethyl-5-methylbarbituric acid, 1-ethyl-5-isobutylbarbituric acid, 1,3-diethyl-5-butylbarbituric acid, 1-cyclohexyl-5-methylbarbituric acid, 1-cyclohexyl-5-ethylbarbituric acid, 1-cyclohexyl-5-octylbarbituric acid, 1-cyclohexyl-5-hexylbarbituric acid, 5-butyl-1-cyclohexylbarbituric acid, thiobarbituric acid, and salts of these (particularly preferred are alkali metal salts or alkali earth metal salts). Examples of the salts of these barbituric acids include sodium 5-butylbarbiturate, sodium 1,3,5-trimethylbarbiturate, and sodium 1-cyclohexyl-5-ethylbarbiturate.

Particularly preferred as barbituric acid compounds are, for example, 5-butylbarbituric acid, 1,3,5-trimethylbarbituric acid, 1-cyclohexyl-5-ethylbarbituric acid, 1-benzyl-5-phenylbarbituric acid, and sodium salts of these barbituric acids.

Examples of the triazine compounds include 2,4,6-tris(trichloromethyl)-s-triazine, 2,4,6-tris(tribromomethyl)-s-triazine, 2-methyl-4,6-bis(trichloromethyl)-s-triazine, 2-methyl-4,6-bis(tribromomethyl)-s-triazine, 2-phenyl-4,6-bis(trichloromethyl)-s-triazine, 2-(p-methoxyphenyl)-4,6-bis(trichloromethyl)-s-triazine, 2-(p-methylthiophenyl)-4,6-bis(trichloromethyl)-s-triazine, 2-(p-chlorophenyl)-4,6-bis(trichloromethyl)-s-triazine, 2-(2,4-dichlorophenyl)-4,6-bis(trichloromethyl)-s-triazine, 2-(p-bromophenyl)-4,6-bis(trichloromethyl)-s-triazine, 2-(p-tolyl)-4,6-bis(trichloromethyl)-s-triazine, 2-n-propyl-4,6-bis(trichloromethyl)-s-triazine, 2-($\alpha,\alpha,\beta$-trichloroethyl)-4,6-bis(trichloromethyl)-s-triazine, 2-styryl-4,6-bis(trichloromethyl)-s-triazine, 2-[2-(p-methoxyphenyl)ethenyl]-4,6-bis(trichloromethyl)-s-triazine, 2-[2-(o-methoxyphenyl)ethenyl]-4,6-bis(trichloromethyl)-s-triazine, 2-[2-(p-butoxyphenyl)ethenyl]-4,6-bis(trichloromethyl)-s-triazine, 2-[2-(3,4-dimethoxyphenyl)ethenyl]-4,6-bis(trichloromethyl)-s-triazine, 2-[2-(3,4,5-trimethoxyphenyl)ethenyl]-4,6-bis(trichloromethyl)-s-triazine, 2-(1-naphthyl)-4,6-bis(trichloromethyl)-s-triazine, 2-(4-biphenylyl)-4,6-bis(trichloromethyl)-s-triazine, 2-[2-{N, N-bis(2-hydroxyethyl)am ino}ethoxy]-4,6-bis(trichloromethyl)-s-triazine, 2-[2-{N-hydroxyethyl-N-ethylam ino}ethoxy]-4,6-bis(trichloromethyl)-s-triazine, 2-[2-{N-hydroxyethyl-N-methylamino}ethoxy]-4,6-bis(trichloromethyl)-s-triazine, and 2-[2-{N, N-dial lylam ino}ethoxy]-4,6-bis(trichloromethyl)-s-triazine.

In view of polymerization activity, preferred among these triazine compounds is 2,4,6-tris(trichloromethyl)-s-triazine. In view of storage stability, preferred are 2-phenyl-4,6-bis(trichloromethyl)-s-triazine, 2-(p-chlorophenyl)-4,6-bis(trichloromethyl)-s-triazine, and 2-(4-biphenylyl)-4,6-bis(trichloromethyl)-s-triazine. The triazine compounds may be used alone, or two or more thereof may be used in combination.

Preferred for use as copper compounds are, for example, copper acetylacetonate, copper(II) acetate, copper oleate, copper(II) chloride, and copper(II) bromide.

Examples of the tin compounds include di-n-butyltin dimaleate, di-n-octyltin dimaleate, di-n-octyltin dilaurate, and di-n-butyltin dilaurate. Particularly preferred as tin compounds are di-n-octyltin dilaurate and di-n-butyltin dilaurate.

The vanadium compounds are preferably vanadium compounds with valences of IV and/or V. Examples of vanadium compounds with valences of IV and/or V include compounds mentioned in JP 2003-96122 A, for example, such as vanadium(IV) oxide, vanadium(IV)oxy acetylacetonate, vanadyl oxalate, vanadyl sulfate, vanadium(IV) oxobis(1-phenyl-1,3-butanedionate), bis(maltolato)oxovanadium(IV), vanadium(V) oxide, sodium metavanadate, and ammonium metavanadate.

Preferred for use as halogen compounds are, for example, dilauryldimethylammonium chloride, lauryldimethylbenzylammonium chloride, benzyltrimethylammonium chloride, tetramethylammonium chloride, benzyldimethylcetylammonium chloride, and dilauryldimethylammonium bromide.

Examples of the aldehydes include terephthalaldehyde, and derivatives of benzaldehyde. Examples of derivatives of benzaldehyde include dimethylaminobenzaldehyde, p-methoxybenzaldehyde, p-ethoxybenzaldehyde, and p-n-octyloxybenzaldehyde. In view of curability, preferred for use is p-n-octyloxybenzaldehyde.

Examples of the thiol compounds include 3-mercaptopropyltrimethoxysilane, 2-mercaptobenzoxazole, decanethiol, and thiobenzoic acid.

Examples of the sulfites include sodium sulfite, potassium sulfite, calcium sulfite, and ammonium sulfite.

Examples of the bisulfites include sodium bisulfite and potassium bisulfite.

Examples of the thiourea compounds include 1-(2-pyridyl)-2-thiourea, thiourea, methylthiourea, ethylthiourea, N,N'-dimethylthiourea, N,N'-diethylthiourea, N,N'-di-n-propylthiourea, N,N'-dicyclohexylthiourea, trimethylthiourea, triethylthiourea, tri-n-propylthiourea, tricyclohexylthiourea, tetramethylthiourea, tetraethylthiourea, tetra-n-propylthiourea, and tetracyclohexylthiourea.

Examples of the aryliodonium salt compounds include salts formed by cations such as diphenyliodonium, bis(p-chlorophenyl)iodonium, di-p-tolyliodonium, bis(p-methoxyphenyl)iodonium, bis(p-tert-butylphenyl)iodonium, p-isopropylphenyl-p-methylphenyliodonium, bis(m-nitrophenyl)iodonium, p-tert-butylphenylphenyliodonium, p-methoxyphenylphenyliodonium, p-octyloxyphenylphenyliodonium, and p-phenoxyphenylphenyliodonium, combined with anions such as nitrate, acetate, chloroacetate, carboxylate, and phenolate.

Examples of the sulfonium salt compounds include salts formed by cations such as dimethylphenacylsulfonium, dimethylbenzylsulfonium, dimethyl-4-hydroxyphenylsulfonium, dimethyl-4-hydroxynaphthylsulfonium, dimethyl-4,7-dihydroxynaphthylsulfonium, dimethyl-4,8-dihydroxynaphthylsulfonium, triphenylsulfonium, p-tolyldiphenylsulfonium, p-tert-butylphenyldiphenylsulfonium, and diphenyl-4-phenylthiophenylsulfonium, combined with anions such as chloride, bromide, p-toluenesulfonate, trifluoromethanesulfonate, tetrafluoroborate, tetrakispentafluorophenylborate, tetrakispentafluorophenylgallate, hexafluorophosphate, hexafluoroarsenate, and hexafluoroantimonate.

Specific examples of the sulfonic acid ester compounds include benzoin tosylate, $\alpha$-methylolbenzoin tosylate, o-nitrobenzyl p-toluenesulfonate, and p-nitrobenzyl-9,10-diethoxyanthracene-2-sulfonate.

When a dental curable composition of the present invention comprises the polymerization accelerator (F), the content of polymerization accelerator (F) is not particularly limited. However, in view of considerations such as curability of the dental curable composition obtained, the content of polymerization accelerator (F) is preferably 0.001 parts or more by mass, more preferably 0.01 parts or more by mass, even more preferably 0.02 parts or more by mass, or may be 0.03 parts or more by mass, 0.05 parts or more by mass, or 0.1 parts or more by mass, relative to 100 parts by mass of the total mass of polymerizable monomer (A). For considerations such as possible precipitation from the dental curable composition when the content of polymerization accelerator (F) is too high, the content of polymerization accelerator (F) is preferably 30 parts or less by mass, more preferably 20 parts or less by mass, even more preferably 10 parts or less by mass, particularly preferably 5 parts or less by mass, or may be 2 parts or less by mass, 1 part or less by mass, or 0.5 parts or less by mass, relative to 100 parts by mass of the total mass of polymerizable monomer (A).

[Additive (G)]

A dental curable composition of the present invention may optionally comprise an additive (G), for example, such as a polymerization inhibitor, a chain transfer agent, an ultraviolet absorber, a fluorescent agent, an antioxidant, an antimicrobial agent, a dispersant, and a pH adjuster, other than the polymerizable monomer (A), polymerization initiator (B), filler (C), colorant (D), polymer (E), and polymerization accelerator (F) described above. The additive (G) may be used alone, or two or more thereof may be used in combination.

Examples of the polymerization inhibitor include 3,5-di-t-butyl-4-hydroxytoluene, hydroquinone, dibutyl hydroquinone, dibutyl hydroquinone monomethyl ether, hydroquinone monomethyl ether, and 2,6-di-t-butylphenol. These may be used alone, or two or more thereof may be used in combination.

Examples of the ultraviolet absorber include benzotriazole compounds such as 2-(2-hydroxyphenyl)benzotriazole, 2-(2-hydroxy-5-methylphenyl)benzotriazole, 2-(2-hydroxy-5-ethylphenyl)benzotriazole, 2-(2-hydroxy-5-propylphenyl)benzotriazole, 2-(2-hydroxy-5-tert-octylphenyl)benzotriazole, and 2-(2-hydroxy-3-tert-butyl-5-methylphenyl)-5-chloro-2H-benzotriazole (Tinuvin 326); and benzoimidazole compounds. These may be used alone, or two or more thereof may be used in combination.

Preferred for use as fluorescent agents are, for example, the compounds disclosed in WO2021/125246. These may be used alone, or two or more thereof may be used in combination.

<<Method of Production of Dental Curable Composition>>

The method of preparation of a dental curable composition of the present invention is not particularly limited, and a dental curable composition of the present invention can be obtained by adding each component to achieve the predetermined content. The order in which components are added is not particularly limited, and the components may be added at once, or may be added in two or more separate portions. In view of enabling the production of a more uniform dental curable composition with improved properties such as mechanical strength, the polymerization initiator (B), polymer (E), polymerization accelerator (F), and additive(G) may be uniformly dissolved in polymerizable monomer (A) in advance. For more uniform dissolution, these may be dissolved at temperatures higher than ordinary temperature, as long as it remains within the effective range of the present invention.

A kneading process may be optionally provided to achieve uniform mixing of filler (C) with the composition containing polymerizable monomer (A). In the kneading process, the composition containing polymerizable monomer (A) is introduced into a kneader with the filler (C), and these are kneaded to prepare a paste-like composition. In the kneading process, the method of kneading is not particularly limited, and known kneading methods can be used, as long as the present invention can exhibit its effects. However, in view of reducing kneading time and preventing variation in the paste, it is preferable to apply heat while kneading. The kneading temperature is preferably 30 to 60°C. A kneading temperature of 30°C or more provides an effect that sufficiently reduces kneading time. With a kneading temperature of 60°C or less, it is possible to inhibit polymerization and curing during kneading. The kneading time is not particularly limited. However, in view of enabling the production of a uniform dental curable composition, the kneading time is preferably 30 minutes to 8 hours. Optionally, vacuum defoaming may be performed while kneading. Here, the vacuum level is not particularly limited. However, it is preferably 5 to 200 Torr for efficient removal of bubbles.

Additionally, a defoaming process, such as vacuum defoaming, may be provided to defoam the paste-like composition that has incorporated the components. In the defoaming process, the paste-like composition is defoamed by extruding it from the defoaming chamber under applied pressure while removing bubbles from the paste inside the defoaming chamber under reduced pressure. While the defoaming conditions are not particularly limited, a vacuum level of 5 to 200 Torr is preferred to efficiently remove bubbles and to reduce uneven distribution of the filler (C) uniformly dispersed in the composition containing polymerizable monomer (A). Concerning defoaming conditions, the duration under reduced pressure is preferably 3 to 30 minutes. The extrusion pressure is preferably 0.5 to 5 MPa. The duration under extrusion pressure is preferably 3 to 30 minutes. Optionally, a heat treatment may be carried out during defoaming. Here, the temperature is not particularly limited. However, a temperature of 40 to 60°C is preferred for efficient removal of bubbles.

The resulting dental curable composition may be filled into a container (such as a syringe), for example.

«Uses»

A dental curable composition of the present invention is not limited to particular uses, and may be used as a variety of dental materials. Specifically, a dental curable composition of the present invention can be suitably used in applications, for example, such as dental composite resins (for example, composite resins for filling cavities in caries, composite resins for abutment construction, composite resins for dental caps, self-adhesive composite resins), denture base resins, denture base liners, impression materials, luting materials (for example, resin cements, resin-added glass ionomer cements), dental bonding agents (for example, orthodontic adhesives, adhesives for application to cavities), tooth fissure sealants, resin blocks for CAD/CAM, temporary crowns, and artificial teeth materials. Because of high aesthetics and superior mechanical strength, a dental curable composition of the present invention is particularly suited for dental composite resins, and resin blocks for CAD/CAM.

The present invention encompasses embodiments combining all or part of the foregoing features, provided that the present invention can exhibit its effects with such combinations made in various forms within the technical idea of the present invention.

EXAMPLES

The following describes the present invention in greater detail by way of Examples and Comparative Examples. It is to be noted, however, that the present invention is not limited to the following EXAMPLES. Details are summarized below, including the test methods and materials used in EXAMPLES.

«Test Method»

[Average Particle Diameter of Filler]

The fillers listed below were measured for average particle diameter by taking an electron micrograph using a scanning electron microscope (SU3500, manufactured by Hitachi, Ltd.). The captured image was transferred to image-analyzing particle size distribution measurement software Mac-View (manufactured by Mountech Co., Ltd.), and the average particle diameter was determined by averaging the particle diameters of randomly selected 100 particles. For non-spherical particles, the particle diameter is defined as the arithmetic mean value of the maximum and minimum lengths of particles.

[Refractive Index of Filler]

For the fillers listed below, a dispersion liquid was prepared by dispersing a certain quantity (0.1 g/mL) of each filler in an organic solvent of a known refractive index (the refractive index was adjusted by mixing two or more organic solvents selected from the group consisting of 1-bromonaphthalene, methyl salicylate, dimethyl formamide, and 1-pentanol). The refractive index (nD25) of the organic solvent that gave the highest light transmission at 589 nm through the dispersion liquid was then determined as the refractive index of the filler at 25°C.

The type of organic solvent with a known refractive index can be selected based on the expected refractive index of the filler, depending on the type of filler being measured for refractive index.

The refractive index of the organic solvent is measured using an Abbe refractometer (manufactured by Atago Co., Ltd. under the trade name NAR-1T LIQUID) with a Na-D light source. The light transmission through the dispersion liquid was measured in a 10 mm path-length quartz cell using an ultraviolet-visible spectrophotometer (manufactured by Shimadzu Corporation under the trade name UV-2400).

[Refractive Index of Polymerizable Monomer (A)]

A uniform mixture containing polymerizable monomer was prepared by mixing polymerizable monomer (A), polymerization initiator (B), polymerization accelerator (F), and additive (G) in the proportions specified in Table 2 for each Example and Comparative Example presented in Table 2. The refractive index of this polymerizable monomer-containing mixture was then measured in a thermostatic chamber at 25°C, using the Abbe refractometer and the method described in [Refractive Index of Filler]. The measured value was determined as the refractive index of polymerizable monomer (A) at 25°C.

In each Example and Comparative Example, when polymer (E) was used, a uniform mixture was prepared by stirring the polymer (E) and the components of the polymerizable monomer-containing mixture (including polymerizable monomer (A)) at 60°C in the proportions specified in Table 2. The refractive index of this uniform mixture was then measured in a thermostatic chamber at 25°C as the refractive index of polymerizable monomer (A) at 25°C.

[Refractive Index of Cured Product (P) of Polymerizable Monomer (A)]

The mixture prepared for the measurement in [Refractive Index of Polymerizable Monomer (A)] was placed in a mold with a hole measuring 10 mm in diameter × 1.0 mm. After placing glass slides to both sides under applied pressure, the sample was cured by exposing both sides to light, for 45 seconds per side, using an LED photopolymerizer ($\alpha$ Light V, manufactured by J. Morita Corp., wavelength: 400 to 408 nm, 465 to 475 nm). The resulting cured product of the sample was removed from the mold, and measured with the Abbe refractometer described in [Refractive Index of Filler]. When setting the cured product on the Abbe refractometer, a solvent (1-bromonaphthalene) having a higher refractive index than the sample and that does not dissolve the sample was dropped on the sample to ensure close contact between the cured product and the measurement surface.

[Metal (M) Content in Filler]

The content of the metal (M) in the fillers was determined by observing the fillers with a scanning electron microscope (SU3500 manufactured by Hitachi, Ltd.) and analyzing the filler elements using the energy-dispersive X-ray analyzer (EX-370, X-Max20, manufactured by Horiba Ltd.) attached to the microscope. The metal (M) content was calculated using the following formula (n = 3).

Content of metal (M) in filler (mass%) = (mass of metallic elements in the filler)/ (mass of all elements in the filler) × 100

[Light Diffusivity LD]

The light diffusivity LD of the cured product was evaluated with a goniophotometer (GP-200 manufactured by Murakami Color Research Laboratory Co., Ltd.). Specifically, the dental curable composition was placed and pressed between cover glasses from top and bottom with 0.25 mm-thick stainless-steel spacers, and cured to prepare a cured sample plate (30 mm in diameter, 0.25 mm thick) by applying light from both sides, for 45 seconds each side, using an LED photopolymerizer ($\alpha$ Light V manufactured by J. Morita Corp.; wavelength: 400 to 408 nm, 465 to 475 nm). The cured plate was then measured for the luminous intensity distribution of transmitted light from -90° to +90° relative to an incident light angle of 0°, using a goniophotometer. The light diffusivity LD was calculated following the formula [2] described above (n = 1).

[Evaluation of Color Compatibility with Artificial Tooth Cavities]

The color compatibility of the dental curable composition for Class II 4 mm cavities was assessed by filling and restoring cavities created in artificial teeth, and measuring the restored area with a dental colorimeter.

Specifically, before forming a Class II cavity, an image of artificial molar No. 6 (Zen Opal molar, Form Number (Mandible): PL16, shade: A1 and A4, manufactured by GC JAPAN) was captured with a dental colorimeter (trade name Crystaleye CE100-DC/JP, analysis software Crystaleye, manufactured by Olympus Corporation). The measurement of the artificial tooth was carried out inside the dark box (check box, top cover) included in the dental colorimeter.

Subsequently, a Class II cavity (4 mm deep), as defined by the dotted lines in FIGS. 1 and 2, was formed in the artificial tooth of each shade (A1 and A4). The area inside the dotted lines in FIGS. 1 and 2 represents the Class II cavity.

The color compatibility was measured at two locations in the Class II cavity: the central portion (the central area on the upper bottom side of the molar, avoiding the pits and fissures), and the proximal portion (within 3 mm from the cavity wall; the side facing the neighboring tooth of the molar). As indicated in FIG. 1, a distance of at least 3 mm was provided between the central portion and the proximal portion.

For the prepared cavity, a dental etchant (K-Etchant GEL, manufactured by Kuraray Noritake Dental Inc.) was used to etch the cavity surface, following the method recommended by the manufacturer (as outlined in the package insert). Specifically, the etchant was applied to the cavity surface with a small brush, left for 40 seconds, and rinsed off with water before drying the cavity surface.

Following this, a dental adhesive (Clearfil® Universal Bond Quick ER, manufactured by Kuraray Noritake Dental Inc.) and a dental ceramic adhesive material (Clearfil® Porcelain Bond Activator, manufactured by Kuraray Noritake Dental Inc.) were mixed in equal amounts. This mixture was used for bonding the cavity surface, following the method recommended by the manufacturer (as outlined in the package insert). Specifically, the equal volume mixture was applied to the cavity surface, air-blown to remove evaporative components, and irradiated with light for 10 seconds using a dental visible light irradiator (PenCure 2000, manufactured by J. Morita Corp.) in normal mode.

Subsequently, the dental curable composition under evaluation was filled into the Class II cavity at once. An artificial tooth impression, prepared beforehand with a silicone impression material (dental occlusal stamping material Memosil 2 manufactured by KULZER JAPAN under this trade name), was then pressed against the dental curable composition to give it the shape of the artificial tooth. Finally, the dental curable composition was exposed to light for 10 seconds in normal mode over the impression, using the dental visible light irradiator. After removing the impression, the dental curable composition was irradiated for 20 seconds in normal mode, resulting in an artificial tooth sample with a filling.

The filled artificial tooth sample was photographed with the dental calorimeter used to capture an image before Class II cavity formation, using the same technique.

Using the accessory analysis software, lightness ($L^*_1$) and chromaticity ($a^*_1$, $b^*_1$) were measured at two locations in the filled area of the sample in its captured image (the areas inside the boxes (central and proximal portions) in FIGS. 1 and 2) in a measurement mode with a 7 band LED light source, according to the L*a*b* evaluation system (JIS Z 8781-4:2013 Color Measurements - Part 4: CIE 1976 L*a*b* color space).

Additionally, lightness ($L^*o$) and chromaticity ($a^*o$, $b^*o$) were measured for the previously photographed untreated artificial teeth (before Class II cavity formation) at the same locations as those in the captured image of the artificial tooth sample with a filling.

From these images, the color difference ΔE* was calculated as an index of color compatibility, using the following formula (n = 1).

$$\Delta E^* = ((L^*_1 - L^*_0)^2 + (a^*_1 - a^*_0)^2 + (b^*_1 - b^*_0)^2)^{1/2}$$

where $L^*_1$, $a^*_1$, and $b^*_1$ represent the lightness (L value) and chromaticity (a value and b value) in the measured portion (central portion or proximal portion) after the dental curable composition is filled in bulk and cured in the Class II cavities, and $L^*_0$, $a^*_0$, and $b^*_0$ represent the lightness (L value) and chromaticity (a value and b value) in the measured portion (central portion or proximal portion) of untreated artificial molars before Class II cavity formation as measured at the same locations as for $L^*_1$, $a^*_1$, and $b^*_1$.

In the evaluation of color compatibility, a ΔE* value of 6.0 or less was deemed satisfactory for both the central and proximal portions of artificial teeth in A1 and A4 shades. The preferred value of ΔE* is 5.5 or less, more preferably 5.0 or less, even more preferably 4.8 or less.

[Measurement of Form Retention]

A glass cylinder with an inner diameter of 8 mm and a height of 50 mm, open at the both ends, was prepared. Also prepared was a cylindrical rubber stopper, 8 mm in diameter and 8 mm in height, which fits inside the cylinder, along with a circular polyester film with a diameter of 8 mm.

The rubber stopper was pushed 10 mm from the bottom opening of the cylinder to create a cylindrical space with a diameter of 8 mm and a height of 10 mm, and the polyester film was inserted into this space. The dental curable composition under evaluation was then filled into the space inside the cylinder with no gaps. Any excess dental curable composition protruding from the top opening of the cylinder was scraped off.

Subsequently, a glass plate, separately prepared in advance, was placed on a table. With the scraped surface of the dental curable composition facing the glass plate, the rubber stopper was pushed out from the other end of the cylinder, expelling the dental curable composition from the cylinder. This produced a cylindrical molded body of the dental curable composition, measuring 8 mm in diameter and 10 mm in height, on the glass plate.

The cylindrical molded body of the dental curable composition, together with the glass plate, was quickly transferred into an open chamber (Model OTC2D manufactured by Yamato Scientific Co., Ltd., thermostatic chamber) that had been set to 37°C. After being left undisturbed for 1 minute, the cylinder was removed from the open chamber, and the height (h [mm]) of the cylindrical molded body of the dental curable composition was measured. The form retention was calculated by dividing h by 100 and multiplying it by 100 ((h/10) × 100 [%]). The measurement was conducted under a yellow lamp to prevent light curing of the dental curable composition (n = 2).

[Visual Assessment of Color Compatibility with Human Tooth Cavities]

Class II cavities, 4 mm deep, were created in two types of extracted human molars, one light-colored and the other dark-colored. A dental adhesive (Clearfil® Universal Bond Quick ER, manufactured by Kuraray Noritake Dental Inc.) was then applied to the surfaces of these cavities, following the method recommended by the manufacturer (as outlined in the package insert).

Subsequently, the dental curable composition under evaluation was filled into the cavities, and shaped to create an occlusal surface using a plugger (double-ended plugger, manufactured by Seto Seisakusho and sold by Nippon Shikakogyosha Co., Ltd., #4).

Once filled, the dental curable composition was cured by exposing it to light for 20 seconds using a dental visible light irradiator (PenCure 2000, manufactured by J. Morita Corp.) in standard mode.

The surface of the filled portion was polished at 5,000 rpm under running water until a glossy finish was achieved, using a polishing point (CompoMaster CA, dental abrasive material (dental abrasive made of rubber), manufactured by Shofu Inc.).

Once polished, the filled portion was evaluated according to the following criteria (n = 1).

A: The shade of the filled portion blends well with the human teeth in both cavities, making it nearly undistinguishable.
B: In at least one of the cavities, the shade of the filled portion looks slightly different from the human teeth, and the filled portion becomes noticeable upon closer inspection.
C: In both cavities, the shade of the filled portion completely differs from the human teeth, making the filled portion noticeable at a glance.

[Evaluation of Handling Properties]

Using a metal dental plugger (condenser #4, #5, manufactured by Yoshida Dental Trade Distribution Co., Ltd.), the composition was filled into a plastic Class II cavity model (Class 2 MO cavity, A55AN-364, manufactured by Nissin Dental Products Inc.) inside an open chamber (Model OTC2D, thermostatic chamber, manufactured by Yamato Scientific Co., Ltd.) at 37°C, and was shaped to create an occlusal surface. During this procedure, the perceived stickiness and ease of shaping were assessed according to the following criteria (n = 1).

A: No stickiness is experienced, and shaping is ease to achieve
B: Some stickiness is present, and/or shaping is somewhat difficult
C: There is serious stickiness and/or difficulty in shaping

[Measurement of Depth of Cure]

The depth of cure was assessed according to ISO 4049:2019, specifically as follows. The dental curable composition was filled into a stainless-steel mold (thickness 12 mm, diameter 4 mm). Subsequently, a film and a glass slide were placed on each side of the mold in this order, and the layers were pressed together. After removing the glass slides, the mold was placed on white filter paper, and the dental curable composition was cured by exposing it to light for 20 seconds from the opposite side using a dental visible light irradiator (PenCure 2000, manufactured by J. Morita Corp.) in standard mode.

After taking out the cured product from the mold, the uncured portions were removed from the cured product, along with the films. The length from the exposed surface was then measured with a micrometer (No. 293-230 manufactured by Mitsutoyo Corporation), and half of this measured length was determined as the depth of cure (n = 3). The average of these values was then calculated.

«Materials»

(Polymerizable Monomer (A))

POBMA: m-phenoxybenzyl methacrylate
3G: triethylene glycol dimethacrylate
DD: 1,10-decanediol dimethacrylate
UDMA: 2,2,4-trimethylhexamethylenebis(2-carbamoyloxyethyl)dimethacrylate
D2.6E: 2,2-bis(4-methacryloyloxypolyethoxyphenyl)propane (with an average of 2.6 moles of ethyleneoxy group added)
Bis-GMA: 2,2-bis[4-(2-hydroxy-3-methacryloyloxypropoxy)phenyl]propane

(Polymerization Initiator (B))

CQ: camphorquinone
TMDPO: 2,4,6-trimethylbenzoyldiphenylphosphine oxide

(Filler (C))

The fillers obtained in the Production Examples below were used. In addition, commercially available products were used without modification, and their properties were assessed as part of Production Examples.

(Filler (C1))

[Production Example 1-1(C1-AL)]

A three-neck flask was charged with 100 parts by mass of commercially available alumina fine particles (AEROXIDE® AluC manufactured by Nippon Aerosil Co., Ltd.), 1,000 parts by mass of toluene, 9 parts by mass of 10-methacryloyloxydecyldihydrogen phosphate, and 9 parts by mass of 11-methacryloyloxyundecyltrimethoxysilane, and these were stirred at 80°C for 3 hours. After removing toluene through distillation under reduced pressure, the mixture was dried in vacuum at 40°C for 16 hours, followed by 3 hours of heating at 90°C to yield inorganic fine particles (C1-AL) with a surface treatment layer. The inorganic fine particles (C1-AL) had an average particle diameter of 20 nm, a refractive index of 1.65, and an aluminum content of 36.0 mass%.

[Production Example 1-2 (C1-BA)]

Using a thermal plasma device (manufactured by Nissei Engineering Inc.), composite oxide particles were produced following the method described in Production Example 1 of composite oxide particles in JP 2019-26504 A, and the method in Surface Treatment Example 1 of composite oxide particles described in this publication. After production, the composite oxide particles were subjected to surface treatment to yield inorganic fine particles (C1-BA) with a surface treatment layer. The inorganic fine particles (C1-BA) had an average particle diameter of 50 nm, a refractive index of 1.54, and a barium content of 15.8 mass%.

(Filler (C2))

[Production Example 1-3 (C2-IC)]

A paste was prepared by adding and mixing 400 parts by mass of commercially available surface-treated barium glass (GM27884 NanoFine 180, surface-treated with 11% 3-methacryloyloxypropyltrimethoxysilane, manufactured by Schott, average particle diameter 0.2 $\mu$m) into 100 parts by mass of a polymerizable monomer-containing composition of Bis-GMA and 3G (the mass ratio of Bis-GMA and 3G = 1:1) with 1 mass% of benzoyl peroxide previously dissolved as a polymerization initiator. Subsequently, the paste underwent thermal polymerization at 100°C for 5 hours in a reduced pressure atmosphere.

The resulting product of polymerization and curing was pulverized with a vibration ball mill until it had an average particle diameter of approximately 5 $\mu$m. Subsequently, 100 parts by mass of this powder was introduced into an aqueous solution of 140 parts by mass of distilled water, 3 parts by mass of 3-methacryloyloxypropyltrimethoxysilane, and 0.15 parts by mass of acetic acid. The mixture was then stirred at 25°C for 2 hours to obtain a slurry. After freezing in a -20°C freezer, the slurry was vacuum dried to remove moisture, and heated at 90°C for 3 hours to yield an organic-inorganic composite filler (C2-IC) with a surface treatment layer. The organic-inorganic composite filler (C2-IC) had an average particle diameter of 5.2 $\mu$m, a refractive index of 1.53, a barium content of 13.9 mass%, and an aluminum content of 3.1 mass%.

[Production Example 1-4 (C2-ZR)]

Inorganic agglomerated particles (C2-ZR) with a surface treatment layer were produced following the method described in Production Example 1 of WO2021/125246. The inorganic agglomerated particles (C2-ZR) had an average particle diameter of 40 nm for the inorganic primary particles, an average particle diameter of 3.0 $\mu$m for the aggregates, a refractive index of 1.53, and a zirconium content of 12.4 mass%.

(Filler (C3))

[Production Example 1-5 (C3-NE)]

A polymerizable monomer-containing composition was obtained by mixing and uniformly dissolving 70 parts by mass of UDMA, 30 parts by mass of DD, and 0.5 parts by mass of benzoyl peroxide. Separately, a surface treatment was carried out for commercially available fumed silica Aerosil OX50 (manufactured by Nippon Aerosil Co., Ltd., average particle diameter: 40 nm) and commercially available fumed silica Aerosil 130 (manufactured by Nippon Aerosil Co., Ltd., average particle diameter: 16 nm) by an ordinary method, using 3-methacryloyloxypropyltrimethoxysilane. A paste-form composition was then obtained by kneading 50 parts by mass of the surface-treated Aerosil OX50, 50 parts by mass of the surface-treated Aerosil 130, and 100 parts by mass of the polymerizable monomer-containing composition. The composition underwent polymerization at 130°C for 3 hours under reduced pressure, and the resulting cured product was pulverized with a vibration ball mill until it had an average particle diameter of approximately 15 $\mu$m. Subsequently, 100 parts by mass of this powder was introduced into an aqueous solution of 200 parts by mass of distilled water, 3 parts by mass of 3-methacryloyloxypropyltrimethoxysilane, and 0.13 parts by mass of acetic acid. The mixture was then stirred at 25°C for 1 hour. After freezing in a -20°C freezer, the slurry was vacuum dried to remove moisture, and heated at 90°C for 3 hours to yield an organic-inorganic composite filler (C3-NE) with a surface treatment layer. The organic-inorganic composite filler (C3-NE) had an average particle diameter of 14.8 $\mu$m, and a refractive index of 1.49. The organic-inorganic composite filler (C3-NE) did not contain metal (M).

[Production Example 1-6 (C3-FM)]

A polymerizable monomer-containing composition was obtained by mixing and uniformly dissolving 70 parts by mass of Bis-GMA, 30 parts by mass of 3G, and 1 part by mass of benzoyl peroxide. Separately, a surface treatment was carried out for commercially available fumed silica Aerosil 50 (manufactured by Nippon Aerosil Co., Ltd., average particle diameter: 0.03 $\mu$m) and commercially available fumed silica Aerosil 130 (manufactured by Nippon Aerosil Co., Ltd., average particle diameter: 16 nm) by an ordinary method, using 3-methacryloyloxypropyltrimethoxysilane.

Thereafter, a paste-form composition was obtained by kneading 50 parts by mass of the surface-treated Aerosil 50, 50 parts by mass of the surface-treated Aerosil 130, and 100 parts by mass of the polymerizable monomer-containing composition. The composition underwent polymerization at 130°C for 3 hours under reduced pressure, and the resulting cured product was pulverized with a vibration ball mill until it had an average particle diameter of approximately 11 $\mu$m. Subsequently, 100 parts by mass of this powder was introduced into an aqueous solution of 200 parts by mass of distilled water, 3 parts by mass of 3-methacryloyloxypropyltrimethoxysilane, and 0.13 parts by mass of acetic acid. The mixture was then stirred at 25°C for 1 hour. After freezing in a -20°C freezer, the slurry was vacuum dried to remove moisture, and heated at 90°C for 3 hours to yield an organic-inorganic composite filler (C3-FM) with a surface treatment layer. The organic-inorganic composite filler (C3-FM) had an average particle diameter of 11.4 $\mu$m, and a refractive index of 1.51. The organic-inorganic composite filler (C3-FM) did not contain metal (M).

[Production Example 1-7 (C3-P5)]

A commercially available aggregated silica (Silica Micro Bead P-500, manufactured by JGC C & C, surface-treated with 3-methacryloyloxypropyltrimethoxysilane) was used without modification (C3-P5). The aggregated silica (C3-P5) had an average particle diameter of 12 nm for the inorganic primary particles, an average particle diameter of 2.0 $\mu$m for the aggregates, and a refractive index of 1.45. The aggregated silica (C3-P5) did not contain metal (M).

(Filler (C4))

[Production Example 1-8 (C4-NF)]

A commercially available barium glass (GM27884 NanoFine 180, manufactured by Schott, surface-treated with 11% 3-methacryloyloxypropyltrimethoxysilane) was used without modification (C4-NF). The barium glass (C4-NF) had an average particle diameter of 0.2 $\mu$m, a refractive index of 1.53, a barium content of 18.0 mass%, and an aluminum content of 3.4 mass%.

[Production Example 1-9 (C4-UF)]

A commercially available barium glass (8235 UF0.7, manufactured by Schott, surface-treated with 4.2% 3-methacryloyloxypropyltrimethoxysilane) was used without modification (C4-UF). The barium glass (C4-UF) had an average particle diameter of 0.7 $\mu$m, a refractive index of 1.55, a barium content of 26.5 mass%, and an aluminum content of 5.3 mass%.

[Production Example 1-10 (C4-YB)]

A commercially available ytterbium fluoride powder (SG-YBF100WSCMP10, manufactured by Sukgyung AT, silica-coated ytterbium fluoride, surface-treated with 10% 3-methacryloyloxypropyltrimethoxysilane) was used without modification (C4-YB). The ytterbium fluoride powder (C4-YB) had an average particle diameter of 0.11 $\mu$m, a refractive index of 1.54, and a ytterbium content of 60.9 mass%.

(Additional Filler (C5))

[Production Example 1-11 (C5-TA)]

To 600 parts by mass of toluene were added 40 parts by mass of 3-methacryloyloxypropyltrimethoxysilane, and 100 parts by mass of commercially available fumed silica Aerosil 130 (manufactured by Nippon Aerosil Co., Ltd., average particle diameter: 16 nm), in this order. These were stirred at 30°C for 30 minutes. The mixture was then placed under reduced pressure for 2 hours under 30°C, Pa conditions to remove toluene from the slurry, using an evaporator. This yielded a silica powder (C5-TA) with a surface treatment layer. The silica powder (C5-TA) had an average particle diameter of 30 nm, and a refractive index of 1.45. The silica powder (C5-TA) did not contain metal (M).

Table 1 below presents the fillers of Production Examples 1-1 to 1-11. The fillers of Production Examples 1-1 to 1-11 did not have a number-based particle size distribution where 90% or more of the particles are within $\pm$5% of the average primary particle diameter, and were not monodisperse.

[Table 1]

|  |  | Metals (M) contained | Metal content (mass%) | Average particle diameter | Refractive index |
|---|---|---|---|---|---|
| Production Example 1-1 | C1-AL | Aluminum | 36.0 | 20 nm | 1.65 |
| Production Example 1-2 | C1-BA | Barium | 15.8 | 50 nm | 1.54 |
| Production Example 1-3 | C2-IC | Barium / Aluminum | 13.9 / 3.1 | 5.2 $\mu$m | 1.53 |
| Production Example 1-4 | C2-ZR | Zirconium | 12.4 | 3.0 $\mu$m | 1.53 |
| Production Example 1-5 | C3-NE | - | - | 14.8 $\mu$m | 1.49 |
| Production Example 1-6 | C3-FM | - | - | 11.4 $\mu$m | 1.51 |
| Production Example 1-7 | C3-P5 | - | - | 2.0 $\mu$m | 1.45 |

(continued)

|  |  | Metals (M) contained | Metal content (mass%) | Average particle diameter | Refractive index |
|---|---|---|---|---|---|
| Production Example 1-8 | C4-NF | Barium / Aluminum | 18.0 / 3.4 | 0.2 $\mu$m | 1.53 |
| Production Example 1-9 | C4-UF | Barium / Aluminum | 26.5 / 5.3 | 0.7 $\mu$m | 1.55 |
| Production Example 1-10 | C4-YB | Ytterbium | 60.9 | 0.11 $\mu$m | 1.54 |
| Production Example 1-11 | C5-TA | - | - | 30 nm | 1.45 |

(Colorant (D))

D-W: white pigment (titanium oxide, rutile-type, refractive index at 25°C: 2.72; manufactured by Fujifilm Wako Pure Chemical Industries, Ltd.)
D-B: black pigment (Toda Color KN-320, manufactured by Toda Kogyo Corp.)
D-Y: yellow pigment (Toda Color TSY-2, manufactured by Toda Kogyo Corp.)
D-R: red pigment (Toda Color 100ED, manufactured by Toda Kogyo Corp.)

(Polymer (E))

[Production Example 2-1 (E-1)]

A three-neck flask was charged with 65 parts by mass of D2.6E, 35 parts by mass of isobornyl methacrylate, and 450 parts by mass of toluene. These were dissolved and subjected to 30 minutes of nitrogen bubbling. After adding 57.5 parts by mass of 2,4-diphenyl-4-methyl-1-pentene and 2.5 parts by mass of benzoyl peroxide, the mixture was stirred under reflux at 80°C using an oil bath. The heating was stopped after 2.5 hours, and the mixture was dropped into 4,000 parts by mass of isopropanol to yield a precipitate. After filtration, the precipitate was thoroughly washed with isopropanol, and vacuum dried overnight at ordinary temperature to yield a white powdery polymer (E-1).
The weight-average molecular weight and the number of unreacted polymerizable functional groups were assessed following the method described in WO2020/122192. The weight-average molecular weight was 48,600, and the number of unreacted polymerizable functional groups was 41 [per molecule].
The weight-average molecular weight is a weight-average molecular weight measured by gel permeation chromatography (GPC) in terms of polystyrene.

(Polymerization Accelerator (F))

PDE: ethyl 4-(N,N-dimethylamino)benzoate

(Additive (G))

TN326: Tinuvin 326 (manufactured by BASF Japan, ultraviolet absorber)
LBL: diethyl 2,5-dihydroxyterephthalate (fluorescent agent)
OBPB: N-[2-(4-oxo-1,3-benzooxazin-2-yl)phenyl]benzenesulfoneamide

(fluorescent agent)

BBN: 1,4-bis(2-benzooxazolyl)naphthalene (fluorescent agent)
BHT: 3,5-di-t-butyl-4-hydroxytoluene (polymerization inhibitor)

[Examples 1 to 11 and Comparative Examples 1 to 4]

The materials of Examples 1 to 11 and Comparative Examples 2 to 4 presented in Tables 2 and 3 were mixed and kneaded in the specified proportions at ordinary temperature (23°C) under a yellow lamp. The resulting uniform mixtures were then defoamed in vacuum to prepare dental curable compositions.

These dental curable compositions were tested using the methods previously described, along with a commercially available composite resin (Omnichroma, manufactured by Tokuyama Dental Corporation; Comparative Example 1). The results are presented in Tables 2 and 3.

[Table 2]

| Components (parts by mass) | | | | Ex. 1 | Ex. 2 | Ex. 3 | Ex. 4 | Ex. 5 | Ex. 6 | Ex. 7 | Ex. 8 | Ex. 9 | Ex. 10 | Ex. 11 |
|---|---|---|---|---|---|---|---|---|---|---|---|---|---|---|
| Polymerizable monomer-containing composition | Polymerizable monomer (A) | | POBMA | 15.0 | 15.0 | 15.0 | 15.0 | 30.0 | | 26.0 | 12.0 | 28.5 | | |
| | | | 3G | 10.0 | 10.0 | 10.0 | 10.0 | 25.0 | 25.0 | 21.6 | 10.0 | | | |
| | | | DD | | | | | | | | | | 10.0 | 10.0 |
| | | | UDMA | 25.0 | 25.0 | 25.0 | 25.0 | 10.0 | 30.0 | 8.6 | 23.0 | 47.5 | | |
| | | | D2.6E | 50.0 | 50.0 | 50.0 | 50.0 | 35.0 | | 30.2 | 50.0 | 19.0 | 40.0 | 40.0 |
| | | | Bis-GMA | | | | | | 45.0 | | | | 50.0 | 50.0 |
| | Polymer (E) | | E-1 | | | | | | | 13.6 | 5.0 | 5.0 | | |
| | Polymerization initiator (B) | | Camphorquinone | 0.5 | 0.5 | 0.5 | 0.5 | 0.5 | 0.5 | 0.5 | 0.5 | 0.5 | 0.4 | 0.4 |
| | | | TMDPO | 0.4 | 0.4 | 0.4 | 0.4 | 0.4 | 0.4 | 0.4 | 0.4 | 0.4 | 0.1 | 0.1 |
| | Polymerization accelerator (F) | | PDE | 0.7 | 0.7 | 0.7 | 0.7 | 0.7 | 0.7 | 0.7 | 0.7 | 0.7 | 0.4 | 0.4 |
| | Additive (G) | | TN326 | 0.5 | 0.5 | 0.5 | 0.5 | 0.5 | 0.5 | 0.5 | 0.5 | 0.5 | 0.3 | 0.3 |
| | | | LBL | 0.05 | | | | 0.05 | 0.05 | 0.05 | 0.05 | 0.05 | 0.05 | 0.05 |
| | | | OBPB | | 0.04 | 0.04 | 0.04 | | | | | | | |
| | | | BBN | | 0.02 | 0.02 | 0.02 | | | | | | | |
| | | | BHT | 0.01 | 0.01 | 0.01 | 0.01 | 0.01 | 0.01 | 0.01 | 0.01 | 0.01 | 0.05 | 0.05 |
| | Total (A)+(B)+(E)+(F)+(G) | | | 2.16 | 2.17 | 2.17 | 2.17 | 2.16 | 2.16 | 2.16 | 2.16 | 2.16 | 1.30 | 1.30 |
| | Refractive index of (A)+(B)+(E)+(F)+(G) mixture | | | 1.520 | 1.520 | 1.520 | 1.520 | 1.520 | 1.508 | 1.525 | 1.522 | 1.520 | 1.537 | 1.537 |
| | Refractive index of cured product (P) | | | 1.554 | 1.554 | 1.554 | 1.554 | 1.561 | 1.538 | 1.561 | 1.553 | 1.553 | 1.561 | 1.561 |

[Table 2] Continued

| Components (parts by mass) | | | | Com. Ex. 1 | Com. Ex. 2 | Com. Ex. 3 | Com. Ex. 4 |
|---|---|---|---|---|---|---|---|
| Polymerizable monomer-containing composition | Polymerizable monomer | (A) | POBMA | Omnichroma | 25.0 | | 15.0 |
| | | | 3G | | 25.0 | 30 | 10.0 |
| | | | DD | | | | |
| | | | UDMA | | 50.0 | 70 | 25.0 |
| | | | D2.6E | | | | 50.0 |
| | | | Bis-GMA | | | | |
| | Polymer | (E) | E-1 | | | | |
| | Polymerization initiator | (B) | Camphorquinone | | 0.5 | 0.5 | 0.5 |
| | | | TMDPO | | 0.4 | 0.4 | 0.4 |
| | Polymerization accelerator | (F) | PDE | | 0.7 | 0.7 | 0.7 |
| | Additive | (G) | TN326 | | 0.5 | 0.5 | 0.5 |
| | | | LBL | | 0.05 | 0.05 | 0.05 |
| | | | OBPB | | | | |
| | | | BBN | | | | |
| | | | BHT | | 0.01 | 0.01 | 0.01 |
| | Total (A)+(B)+(E)+(F)+(G) | | | | 2.16 | 2.16 | 2.16 |
| | Refractive index of (A)+(B)+(E)+(F)+(G) mixture | | | | 1.497 | 1.476 | 1.520 |
| | Refractive index of cured product (P) | | | | 1.537 | 1.511 | 1.554 |

[Table 3]

| Components (parts by mass) | | | | Ex. 1 | Ex. 2 | Ex. 3 | Ex. 4 | Ex. 5 | Ex. 6 | Ex. 7 | Ex. 8 |
|---|---|---|---|---|---|---|---|---|---|---|---|
| Dental curable composition | Polymerizable monomer-containing composition | (A)+(B)+(E)+(F)+(G) | | 20.3 | 20.3 | 20.3 | 20.3 | 19.3 | 20.3 | 20.8 | 20.3 |
| | Filler | (C1) | C1-AL | 40 | 40 | 40 | | 3.0 | 40 | 3.0 | 40 |
| | | | C1-BA | | | | 40 | | | | |
| | | (C2) | C2-IC | 44.2 | | | 442 | 45.2 | 44.2 | 43.7 | 44.2 |
| | | | C2-ZR | | 442 | 422 | | | | | |
| | | (C3) | C3-NE | 40 | 40 | | 40 | 40 | 40 | 40 | 40 |
| | | | C3-FM | | | | | | | | |
| | | | C3-P5 | | | 6.0 | | | | | |
| | | (C4) | C4-NF | 22.5 | 22.5 | 22.5 | 22.5 | 22.5 | 22.5 | 22.5 | 22.5 |
| | | | C4-UF | | | | | | | | |
| | | | C4-YB | 5.0 | 5.0 | 5.0 | 5.0 | 5.0 | 5.0 | 50 | 50 |
| | | (C5) | C5-TA | | | | | 1.0 | | 1.0 | |
| | Total | | | 100.0 | 1000 | 1000 | 1000 | 1000 | 1000 | 1000 | 1000 |
| | Refractive index difference | $0 \leq nC1-nA \leq 0.2$ | | 0.130 | 0.130 | 0.130 | 0.020 | 0.130 | 0.142 | 0.125 | 0.128 |
| | | $-0.03 \leq nC2-nA \leq 0.03$ | | 0.010 | 0.010 | 0.010 | 0.010 | 0.010 | 0.022 | 0.005 | 0.008 |
| | | $-02 \leq nC3-nA \leq 0$ | | -0.030 | -0.030 | -0.070 | -0.030 | -0.030 | -0.018 | -0.035 | -0.032 |
| | | $-0.035 \leq nC4(NF)-nA \leq 0.035$ | | 0.010 | 0.010 | 0.010 | 0.010 | 0.010 | 0.022 | 0.005 | 0.008 |
| | | $-0.035 \leq nC4(UF)-nA \leq 0.035$ | | - | - | - | - | - | - | - | - |
| | | $-0.035 \leq nC4(YB)-nA \leq 0.035$ | | 0.020 | 0.020 | 0.020 | 0.020 | 0.020 | 0.032 | 0.015 | 0.018 |

(continued)

| Components (parts by mass) | | | | Ex. 1 | Ex. 2 | Ex. 3 | Ex. 4 | Ex. 5 | Ex. 6 | Ex. 7 | Ex. 8 |
|---|---|---|---|---|---|---|---|---|---|---|---|
| | Colorant (Relative to 100 parts by mass of polymerizable monomer-containing composition) | (D) | D-W | 0.0350 | 0.0350 | 0.0350 | 0.0350 | 0.0350 | 0.0820 | 0.0350 | 0.0350 |
| | | | D-B | | | | | | | | |
| | | | D-Y | 0.0065 | 0.0065 | 0.0065 | 0.0065 | 0.0065 | 0.0085 | 0.0065 | 0.0065 |
| | | | D-R | 0.0026 | 0.0026 | 0.0026 | 0.0026 | 0.0026 | 0.0030 | 0.0026 | 0.0026 |
| Properties | Color compatibility (4 mm Class II) | $\Delta E^*$ (A1, proximal) | | 4.5 | 4.6 | 4.5 | 4.6 | 3.9 | 4.5 | 4.1 | 4.4 |
| | | $\Delta E^*$ (A1, central) | | 3.6 | 4.3 | 4.4 | 4.2 | 4.2 | 4.6 | 4.5 | 4.4 |
| | | $\Delta E^*$ (A4, proximal) | | 4.7 | 4.5 | 3.8 | 40 | 4.2 | 4.2 | 40 | 4.4 |
| | | $\Delta E^*$ (A4, central) | | 4.8 | 4.7 | 3.6 | 4.7 | 3.4 | 4.5 | 3.4 | 2.8 |
| | Form retention | | | 95% | 95% | 95% | 95% | 100% | 100% | 100% | 100% |
| | Light diffusivity LD | | | 0.22 | 0.22 | 0.0030 | 0.22 | 0.22 | 020 | 0.27 | 0.22 |
| | Color compatibility by visual assessment | | | A | A | A | A | A | A | A | A |
| | Handling properties | | | A | A | A | A | A | A | A | A |
| | Depth of cure (ISO) mm | | | 4.3 | 4.3 | 4.5 | 4.2 | 4.3 | 3.1 | 4.3 | 4.2 |

[Table 3] Continued

| Components (parts by mass) | | | | Ex. 9 | Ex. 10 | Ex. 11 | Com. Ex. 1 | Com. Ex. 2 | Com. Ex. 3 | Com. Ex. 4 |
|---|---|---|---|---|---|---|---|---|---|---|
| Dental curable composition | Polymerizable monomer-containing composition | (A)+(B)+(E)+(F)+(G) | | 19.6 | 23.0 | 23.0 | Omnichroma | 20.3 | 23.0 | 20.3 |
| | Filler | (C1) | C1-AL | 40 | | 3.0 | | 40 | | 40 |
| | | | C1-BA | | | | | | | |
| | | (C2) | C2-IC | 44.9 | | | | 442 | | 442 |
| | | | C2-ZR | | | | | | | |
| | | (C3) | C3-NE | 40 | | | | 40 | | 40 |
| | | | C3-FM | | 160 | 16.0 | | | 16.0 | |
| | | | C3-P5 | | | | | | | |
| | | (C4) | C4-NF | 22.5 | | | | 22.5 | | 22.5 |
| | | | C4-UF | | 580 | 580 | | | 58.0 | |
| | | | C4-YB | 5.0 | | | | 5.0 | | 50 |
| | | (C5) | C5-TA | | 3.0 | | | | 3.0 | |
| | Total | | | 1000 | 1000 | 1000 | | 1000 | 1000 | 1000 |
| | Refractive index difference | $0 \leq nC1-nA \leq 0.2$ | | 0.130 | - | 0.113 | | 0.153 | - | 0.130 |
| | | $-0.03 \leq nC2-nA \leq 0.03$ | | 0.010 | - | - | | 0.033 | - | 0.010 |
| | | $-0.2 \leq nC3-nA \leq 0$ | | -0.030 | -0.027 | -0.027 | | -0.007 | 0.034 | -0.030 |
| | | $-0.035 \leq nC4(NF)-nA \leq 0.035$ | | 0.010 | - | - | | 0.033 | - | 0.010 |
| | | $-0.035 \leq nC4(UF)-nA \leq 0.035$ | | - | 0.013 | 0.013 | | - | 0.074 | - |
| | | $-0.035 \leq nC4(YB)-nA \leq 0.035$ | | 0.020 | - | - | | 0.043 | - | 0.020 |

| Components (parts by mass) | | | | Ex. 9 | Ex. 10 | Ex. 11 | Com. Ex. 1 | Com. Ex. 2 | Com. Ex. 3 | Com. Ex. 4 |
|---|---|---|---|---|---|---|---|---|---|---|
| | Colorant (Relative to 100 parts by mass of polymerizable monomer-containing composition) | (D) | D-W | 0.0230 | 0.0700 | 0.0700 | | 0.0350 | 0.0700 | 0.0700 |
| | | | D-B | 0.0001 | 0.0016 | 0.0016 | | | 0.0016 | |
| | | | D-Y | 0.0500 | 0.0143 | 0.0143 | | 0.0065 | 0.0143 | 0.0130 |
| | | | D-R | 0.0028 | 0.0037 | 0.0037 | | 0.0026 | 0.0037 | 0.0052 |
| Properties | Color compatibility (4 mm Class II) | $\Delta E^*$ (A1, proximal) | | 4.6 | 2.9 | 4.1 | 6.3 | 6.1 | 5.6 | 62 |
| | | $\Delta E^*$ (A1, central) | | 40 | 3.3 | 3.7 | 4.9 | 3.1 | 4.7 | 5.7 |
| | | $\Delta E^*$ (A4, proximal) | | 4.5 | 4.5 | 3.1 | 3.3 | 8.3 | 7.8 | 4.1 |
| | | $\Delta E^*$ (A4, central) | | 3.5 | 4.5 | 3.3 | 5.8 | 9.8 | 7.1 | 6.5 |
| | Form retention | | | 100% | 100% | 100% | 80% | 95% | 85% | 100% |
| | Light diffusivity LD | | | 0.22 | 0.56 | 0.56 | 0 | 0.48 | 0.85 | 0.22 |
| | Color compatibility by visual assessment | | | A | A | A | B | C | C | B |
| | Handling properties | | | A | A | A | C | A | B | A |
| | Depth of cure (ISO) mm | | | 4.3 | 3.0 | 3.7 | 4.2 | 4.1 | 2.8 | 3.5 |

As can be seen in Tables 2 and 3, the dental curable compositions of the present invention show favorable color compatibility for deep Class II cavities of 4 mm depth in human teeth, using a single formulation.

Additionally, the color compatibility for Class I cavities and the central portion of Class II cavities can be seen as being essentially the same. Specifically, the central portion of the Class II cavity indicated by black broken lines in FIG. 1 can be regarded as a Class I cavity because the circle in this central portion schematically represents a Class I cavity. It can be seen from this that the dental curable compositions of the present invention also show favorable color compatibility for deep Class I cavities of 4 mm depth in human teeth, using single formulations.

INDUSTRIAL APPLICABILITY

A dental curable composition of the present invention exhibits favorable color compatibility with a wide range of natural tooth shades using just one formulation, even in deep Class II cavities with a depth of 4 mm. This makes a dental curable composition of the present invention suitable for use in applications such as dental composite resins.

**Claims**

1. A dental curable composition comprising a polymerizable monomer (A), a polymerization initiator (B), and a filler (C), wherein the dental curable composition shows a color difference $\Delta E^*$ of 6.0 or less as calculated by the formula [1] below in both a central portion and a proximal portion of artificial molars of A1 and A4 shades when filled in bulk and cured in Class II cavities of 4.0 mm depth formed in the artificial molars,

$$\Delta E^* = ((L^*_1 - L^*_0)^2 + (a^*_1 - a^*_0)^2 + (b^*_1 - b^*_0)^2)^{1/2} \qquad [1]$$

where $L^*_1$, $a^*_1$, and $b^*_1$ represent the lightness (L value) and chromaticity (a value and b value) in the central or proximal portion after the dental curable composition is filled in bulk and cured in the Class II cavities, and $L^*_0$, $a^*_0$, and $b^*_0$ represent the lightness (L value) and chromaticity (a value and b value) in the central or proximal portion of untreated artificial molars before Class II cavity formation as measured at the same locations as for $L^*_1$, $a^*_1$, and $b^*_1$.

2. The dental curable composition according to claim 1, which has a form retention of 90% or more at 37°C in paste form as calculated by the formula below when formed into a cylindrical molded body with a diameter of 8 mm and a height of 10 mm on a glass plate, and the height h [mm] of the cylindrical molded body is measured after being left to stand at 37°C for 1 minute,

$$\text{form retention (\%)} = (h/10) \times 100$$

3. The dental curable composition according to claim 1 or 2, wherein a 0.25 mm thick sample plate made of a cured product of the dental curable composition satisfies a light diffusivity LD of 0.0001 to 0.75 as defined by the following formula [2],

$$LD = (I_5/\cos 5°)/I_0 \qquad [2]$$

where I represents the luminous intensity of light transmitted through the sample plate, and $I_0$ and $I_5$ represent the luminous intensities of transmitted light at 0- and 5-degree angles, respectively, with respect to a direction perpendicular to the sample plate (the direction of light incidence).

4. The dental curable composition according to any one of claims 1 to 3, which further comprises a colorant (D) (the colorant (D) having a refractive index higher than 2.00 at 25°C when the colorant (D) is an inorganic pigment).

5. The dental curable composition according to any one of claims 1 to 4, wherein the filler (C) comprises at least one filler selected from the group consisting of the following fillers (C1), (C2), (C3), and (C4),

filler (C1): a filler having an average particle diameter of 0.001 $\mu$m or more and less than 0.1 $\mu$m, and containing at least one metal (M) selected from the group consisting of aluminum, titanium, strontium, zirconium, barium, lanthanum, and ytterbium;
filler (C2): a filler having an average particle diameter of 1 $\mu$m or more and 50 $\mu$m or less, and containing 5 mass% or more of the metal (M);

filler (C3): a light-diffusing filler having an average particle diameter of 1 μm or more and 50 μm or less, and containing 0 mass% or more and less than 5 mass% of the metal (M);

filler (C4): a filler having an average particle diameter of 0.1 μm or more and less than 1 μm.

6. The dental curable composition according to claim 5, wherein the filler (C) comprises at least two fillers selected from the group consisting of the fillers (C1), (C2), (C3), and (C4).

7. The dental curable composition according to claim 5 or 6, wherein the filler (C) comprises the filler (C2) and/or the filler (C3).

8. The dental curable composition according to any one of claims 5 to 7, wherein the filler (C) comprises the filler (C1).

9. The dental curable composition according to any one of claims 5 to 7, wherein the filler (C2) is an inorganic agglomerated particle (C2-1) formed by agglomeration of inorganic primary particles (x), or an organic-inorganic composite filler (C2-2) containing inorganic primary particles (x), and the inorganic primary particles (x) have an average particle diameter of 0.001 μm or more and less than 1 μm.

10. The dental curable composition according to any one of claims 5 to 7, wherein the filler (C3) is an inorganic agglomerated particle (C3-1) formed by agglomeration of inorganic primary particles (x), or an organic-inorganic composite filler (C3-2) containing inorganic primary particles (x), and the inorganic primary particles (x) have an average particle diameter of 0.001 μm or more and less than 1 μm.

11. The dental curable composition according to any one of claims 5 to 10, wherein the filler (C) comprises all of the fillers (C1), (C2), (C3), and (C4).

12. The dental curable composition according to any one of claims 5 to 11, wherein the total content of the fillers (C1), (C2), (C3), and (C4) is 10 to 97 mass%.

13. The dental curable composition according to any one of claims 5 to 12, wherein the fillers (C1), (C2), (C3), and (C4) satisfy the following formulae [I-1], [I-2], [I-3], and [I-4], respectively,

$$0 \leq nC1\text{-}nA \leq 0.2 \qquad [\text{I-1}]$$

$$-0.03 \leq nC2\text{-}nA \leq 0.03 \qquad [\text{I-2}]$$

$$-0.2 \leq nC3\text{-}nA \leq 0 \qquad [\text{I-3}]$$

$$-0.035 \leq nC4\text{-}nA \leq 0.035 \qquad [\text{I-4}]$$

where nA represents the refractive index of the polymerizable monomer (A) at 25°C, ranging from 1.35 to 1.70, and nC1, nC2, nC3, and nC4 represent the refractive indices of the fillers (C1), (C2), (C3), and (C4), respectively, at 25°C, ranging from 1.30 to 2.00.

FIG.1

FIG.2

## INTERNATIONAL SEARCH REPORT

| International application No. |
| --- |
| **PCT/JP2023/013748** |

### A. CLASSIFICATION OF SUBJECT MATTER

*A61K 6/884*(2020.01)i; *A61K 6/15*(2020.01)i; *A61K 6/16*(2020.01)i; *A61K 6/17*(2020.01)i; *A61K 6/831*(2020.01)i; *A61K 6/84*(2020.01)i

FI: A61K6/884; A61K6/15; A61K6/16; A61K6/17; A61K6/831; A61K6/84

According to International Patent Classification (IPC) or to both national classification and IPC

### B. FIELDS SEARCHED

Minimum documentation searched (classification system followed by classification symbols)

A61K6/884; A61K6/15; A61K6/16; A61K6/17; A61K6/831; A61K6/84

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| A | WO 2018/194032 A1 (TOKUYAMA DENTAL CORPORATION) 25 October 2018 (2018-10-25) <br> in particular, claims, examples | 1-13 |
| A | WO 2014/050634 A1 (TOKUYAMA DENTAL CORPORATION) 03 April 2014 (2014-04-03) <br> in particular, claims, examples | 1-13 |
| A | JP 2021-84865 A (TOKUYAMA DENTAL CORPORATION) 03 June 2021 (2021-06-03) <br> in particular, claims, examples | 1-13 |
| A | JP 2021-080196 A (KURARAY NORITAKE DENTAL INC) 27 May 2021 (2021-05-27) <br> in particular, claims, examples | 1-13 |
| P, A | WO 2022/092193 A1 (KURARAY NORITAKE DENTAL INC) 05 May 2022 (2022-05-05) <br> in particular, claims, examples | 1-13 |

☐ Further documents are listed in the continuation of Box C.    ☑ See patent family annex.

\* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **07 June 2023** | **20 June 2023** |

| Name and mailing address of the ISA/JP | Authorized officer |
| --- | --- |
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

**INTERNATIONAL SEARCH REPORT**
Information on patent family members

International application No.

**PCT/JP2023/013748**

| Patent document cited in search report | | | Publication date (day/month/year) | Patent family member(s) | Publication date (day/month/year) |
|---|---|---|---|---|---|
| WO | 2018/194032 | A1 | 25 October 2018 | US 2020/0121564 A1<br>in particular, claims, examples<br>EP 3613779 A1<br>CN 110431156 A<br>KR 10-2019-0132998 A | |
| WO | 2014/050634 | A1 | 03 April 2014 | US 2015/0272833 A1<br>in particular, claims, examples<br>EP 2902007 A1<br>EP 3446674 A1 | |
| JP | 2021-84865 | A | 03 June 2021 | (Family: none) | |
| JP | 2021-080196 | A | 27 May 2021 | (Family: none) | |
| WO | 2022/092193 | A1 | 05 May 2022 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2018194032 A
- JP H101473 A
- JP H1192461 A
- JP H093109 A
- JP H10245525 A
- JP S5042696 A
- JP 2011144121 A
- JP 2006510583 T
- WO 2020122192 A
- JP 2003096122 A
- WO 2021125246 A
- JP 2019026504 A

### Non-patent literature cited in the description

- Guidelines for Dental Caries Treatment, 139-152